# EUROPEAN PATENT APPLICATION

(11) **EP 4 489 009 A1**
(43) Date of publication of application: **08.01.2025**
(21) Application number: 23760169.5
(22) Date of filing: 27.02.2023
(51) Int. Cl.: G16B 5/00

(54) **INFERENCE DEVICE, GENERATION DEVICE, INFERENCE PROGRAM, AND GENERATION PROGRAM**

(30) Priority: 28.02.2022 JP 2022028996
(71) Applicant: Takeda Pharmaceutical Company Limited, Osaka-shi, Osaka 541-0045 (JP)
(72) Inventor: MAEDA, Tomohiro, Fujisawa-shi, Kanagawa 251-0012 (JP); OGAWA, Nozomi, Fujisawa-shi, Kanagawa 251-0012 (JP)
(74) Representative: Carpmaels & Ransford LLP
(86) International application number: PCT/JP2023/007046
(87) International publication number: WO 2023/163171

(57) **Abstract**

An operation of designing or selecting a chemical structure of a lipid molecule is supported. An inference device includes an acquisition unit configured to acquire input data including at least chemical structure information on a lipid molecule; an inference unit configured to input the input data newly acquired by the acquisition unit into a learned model generated by performing a learning process on a learning model that associates input data including at least chemical structure information on a lipid molecule with information indicating distribution or behavior of an active ingredient encapsulated in a particle containing the lipid molecule in a living organism, to infer information indicating distribution or behavior in the living organism, associated with the newly acquired input data; and an output unit configured to output, based on the inferred information indicating the distribution or behavior in the living organism, information indicating distribution or behavior in a predetermined tissue and/or cell in the living organism or information indicating behavior in any one or more of tissue, blood, or urine.

## Description

### TECHNICAL FIELD

The present disclosure relates to an inference device, a generation device, and an inference program, and a generation program.

### BACKGROUND

A drug delivery system (DDS) using a particle containing lipid molecules for introducing an active ingredient such as a nucleic acid into a cell with high efficiency is known. In the system, by causing the particle containing lipid molecules to encapsulate an active ingredient, a complex particle is formed, and the active ingredient is delivered to a target tissue or cell via the complex particle.

### Related Art Document

### Patent Document

[Patent Document 1] Japanese Laid-open Patent Application Publication No. 2010-133720

### SUMMARY OF THE INVENTION

### Problem to be solved by the invention

With respect to the above, the design and selection of the chemical structure of the lipid molecule forming the particle encapsulating the active ingredient are generally performed manually, and the design and selection of a chemical structure of an appropriate lipid molecule according to the purpose largely depend on the experience and know-how of a skilled person thereof. Additionally, whether a lipid molecule having a designed or selected chemical structure is delivered to a target tissue or cell, or whether the lipid molecule exerts a desired effect in a target tissue or cell is generally evaluated by performing an experiment.

Furthermore, because the chemical structures are narrowed down by repeating an operation of redesigning and reselecting the lipid molecule according to the evaluation result, it takes a considerable amount of time to search for more appropriate chemical structure of the lipid molecule. In addition, it is difficult to accumulate know-how for designing or selecting chemical structures of various active ingredients and lipid molecules suitable for purposes only by searching for limited types of active ingredients and lipid molecules suitable for purposes.

The present disclosure aims to support an operation of designing or selecting a chemical structure of a lipid molecule forming a particle encapsulating an active ingredient.

### Means for Solving the Problem

In view of the above problems, as a result of eagerly studying the above problem, the present inventors have found that a learning model that is based on chemical structure information on a lipid molecule and information indicating distribution or behavior of an active ingredient in a living organism can be generated, and information indicating distribution or behavior of the active ingredient in the living organism can be inferred by using the learning model.

That is, the present disclosure provides the following.
[1] An inference device including:
   an acquisition unit configured to acquire input data including at least chemical structure information on a lipid molecule;
   an inference unit configured to input the input data newly acquired by the acquisition unit into a learned model generated by performing a learning process on a learning model that associates input data including at least chemical structure information on a lipid molecule with information indicating distribution or behavior of an active ingredient encapsulated in a particle containing the lipid molecule in a living organism, to infer information indicating distribution or behavior in the living organism, associated with the newly acquired input data; and
   an output unit configured to output, based on the inferred information indicating the distribution or behavior in the living organism, information indicating distribution or behavior in a predetermined tissue and/or cell in the living organism or information indicating behavior in any one or more of tissue, blood, or urine.
[2] The inference device as described in [1],
   wherein the information indicating the distribution or behavior in the living organism includes mass distribution data or expression level distribution data of the active ingredient encapsulated in the particle containing the lipid molecule in the living organism at each time,
   wherein the inference unit infers mass distribution data or expression level distribution data at each time, associated with the newly acquired input data, and
   wherein the output unit outputs a change over time in mass or an expression level in a predetermined tissue and/or cell based on the inferred mass distribution data or expression level distribution data at each time.
[3] The inference device as described in [2], wherein the expression level distribution data at each time used when the learning process is performed is fluorescence intensity distribution data at each time captured by introducing, into the living organism, a nucleic acid encoding a fluorescent protein, the nucleic acid being encapsulated in the particle containing the lipid molecule having the chemical structure information used when the learning process is performed.
[4] The inference device as described in [3], wherein the mass distribution data at each time used when the learning process is performed is calculated based on the fluorescence intensity distribution data at each time.
[5] The inference device as described in [2], wherein the mass distribution data at each time used when the learning process is performed is fluorescence intensity distribution data at each time captured by introducing, into the living organism, a nucleic acid labeled with a fluorescent protein, the nucleic acid being encapsulated in the particle containing the lipid molecule having the chemical structure information used when the learning process is performed.
[6] The inference device as described in any one of [3] to [5], wherein the learned model is generated by updating model parameters of the learning model so that output data when the input data including at least the chemical structure information on the lipid molecule is input into the learning model approaches the fluorescence intensity distribution data at each time.
[7] The inference device as described in [2], wherein the output unit calculates the expression level at each time in the predetermined tissue and/or cell in the living organism based on the inferred expression level distribution data at each time, and outputs the change over time in the expression level in the predetermined tissue and/or cell in the living organism by interpolating an expression level between times.
[8] The inference device as described in [2], wherein the output unit calculates the mass at each time in the predetermined tissue and/or cell in the living organism based on the inferred mass distribution data at each time, and outputs the change over time in the mass in the predetermined tissue and/or cell in the living organism by interpolating mass between times.
[9] The inference device as described in [1],
   wherein the information indicating the distribution or behavior in the living organism includes PK data and/or PD data of the active ingredient encapsulated in the particle containing the lipid molecule,
   wherein the inference unit infers PK data and/or PD data associated with the newly acquired input data, and
   wherein the output unit outputs, based on the inferred PK data and/or PD data, a feature related to a change over time in any one or more of a tissue concentration, a blood concentration, or a urine concentration, and a feature indicating a relationship between any one or more of a tissue concentration, a blood concentration, or a urine concentration; and a pharmacological effect and/or toxicity.
[10] The inference device as described in [9], wherein the output unit calculates PK/PD data based on the inferred PK data and PD data, and outputs a feature related to a change over time in the pharmacological effect and/or toxicity based on the calculated PK/PD data.
[11] The inference device as described in [9], wherein the PK data and/or PD data used when the learning process is performed is PK data and/or PD data measured by introducing, into the living organism, the active ingredient encapsulated in the particle containing the lipid molecule having the chemical structure information used when the learning process is performed.
[12] The inference device as described in [11], wherein the learned model is generated by updating model parameters of the learning model so that an output when the input data including at least the chemical structure information on the lipid molecule is input into the learned model approaches the measured PK data and/or PD data.
[13] A generation device that repeats a generation process of generating chemical structure information on a new lipid molecule until the information indicating distribution or behavior in the predetermined tissue and/or cell in the living organism or the information indicating behavior in any one or more of the tissue, blood, or urine, output by the output unit of the inference device as described in any one of [1] to [12], satisfies a predetermined condition.
[14] The generation device as described in [13], wherein the generation device selects a search space from among a plurality of search spaces obtained according to a combination of a molecular fragment of a formable hydrocarbon and a chemical skeleton of a lipid molecule, based on the information indicating the distribution or behavior in the predetermined tissue and/or cell in the living organism or the information indicating the behavior in any one or more of the tissue, blood, or urine, and generates chemical structure information on a next new lipid molecule by using a characteristic of the selected search space.
[15] The generation device as described in [14], wherein the plurality of search spaces are different from each other in a combination of a length, a degree of saturation, and a number of branches of the molecular fragment and a type of the chemical skeleton of the lipid molecule.
[16] The generation device as described in [14], wherein the generation device generates the chemical structure information on the next new lipid molecule under a predetermined constraint condition.
[17] The generation device as described in [16], wherein the generation device generates the chemical structure information on the next new lipid molecule by using a precondition for designing or selecting a chemical structure of the lipid molecule forming the particle including the active ingredient as the predetermined constraint condition.
[18] An inference program for causing a computer to execute:
   an acquisition step of acquiring input data including at least chemical structure information on a lipid molecule;
   an inference step of inputting the input data newly acquired in the acquisition step into a learned model generated by performing a learning process on a learning model that associates input data including at least chemical structure information on a lipid molecule with information indicating distribution or behavior of an active ingredient encapsulated in a particle containing the lipid molecule in a living organism, to infer information indicating distribution or behavior in a living organism, associated with the newly acquired input data; and
   an output step of outputting, based on the inferred information indicating the distribution or behavior in the living organism, information indicating distribution or behavior in a predetermined tissue and/or cell in the living organism or information indicating behavior in any one or more of tissue, blood, or urine.
[19] A generation program for causing a computer to execute a generation step of repeating a generation process of generating chemical structure information on a new lipid molecule until the information indicating the distribution or behavior in the predetermined tissue and/or cell in the living organism or the information indicating the behavior in any one or more of the tissue, blood, or urine, output by the output unit of the inference device as described in any one of [1] to [12], satisfies a predetermined condition.

### Effect of the invention

According to the present disclosure, an operation of designing or selecting a chemical structure of a lipid molecule that forms a particle encapsulating an active ingredient can be supported.

### BRIEF DESCRIPTION OF THE DRAWINGS

[FIG. 1] FIG. 1 is a diagram illustrating an example of collecting expression level distribution data.
[FIG. 2] FIG. 2 is a diagram illustrating an example of collecting PK data and PD data.
[FIG. 3] FIG. 3 is a first diagram for explaining an outline of a process in an evaluation system.
[FIG. 4] FIG. 4 is a diagram illustrating an example of a hardware configuration of a learning device or an inference device.
[FIG. 5] FIG. 5 is a first diagram illustrating an example of a functional configuration of the learning device.
[FIG. 6] FIG. 6 is a first diagram illustrating an example of a functional configuration of the inference device.
[FIG. 7] FIG. 7 is a second diagram illustrating an example of the functional configuration of the learning device.
[FIG. 8] FIG. 8 is a second diagram illustrating an example of the functional configuration of the inference device.
[FIG. 9] FIG. 9 is an example of a first flowchart illustrating a flow of an evaluation process.
[FIG. 10] FIG. 10 is a first diagram illustrating a specific example of training data and verification data.
[FIG. 11] FIG. 11 is a first diagram illustrating a specific example of a molecular descriptor.
[FIG. 12] FIG. 12 is a first diagram illustrating a specific example of a verification result.
[FIG. 13] FIG. 13 is a second diagram illustrating a specific example of the training data and the verification data.
[FIG. 14] FIG. 14 is a second diagram illustrating a specific example of the molecular descriptor.
[FIG. 15] FIG. 15 is a second diagram illustrating a specific example of the verification result.
[FIG. 16] FIG. 16 is a second diagram for explaining an outline of the process in the evaluation system.
[FIG. 17] FIG. 17 is an example of a second flowchart illustrating the flow of the evaluation process.
[FIG. 18] FIG. 18 is an example of a flowchart illustrating a flow of a generation process.
[FIG. 19] FIG. 19 is a first diagram illustrating a specific example of chemical structure information on a generated lipid molecule.
[FIG. 20] FIG. 20 is a second diagram illustrating a specific example of the chemical structure information on the generated lipid molecule.
[FIG. 21] FIG. 21 is a third diagram illustrating an example of the functional configuration of the inference device.
[FIG. 22] FIG. 22 is a diagram illustrating an example of collecting mass distribution data.
[FIG. 23] FIG. 23 is a third diagram illustrating an example of the functional configuration of the learning device.
[FIG. 24] FIG. 24 is a fourth diagram illustrating an example of the functional configuration of the inference device.

### DESCRIPTION OF THE EMBODIMENTS

In the following, each embodiment will be described with reference to the accompanying drawings. Here, in the present specification and drawings, components having substantially the same functional configuration are denoted by the same reference numerals, and duplicated description thereof will be omitted.

### [First Embodiment]

### <Example of Collecting Expression Level Distribution Data>

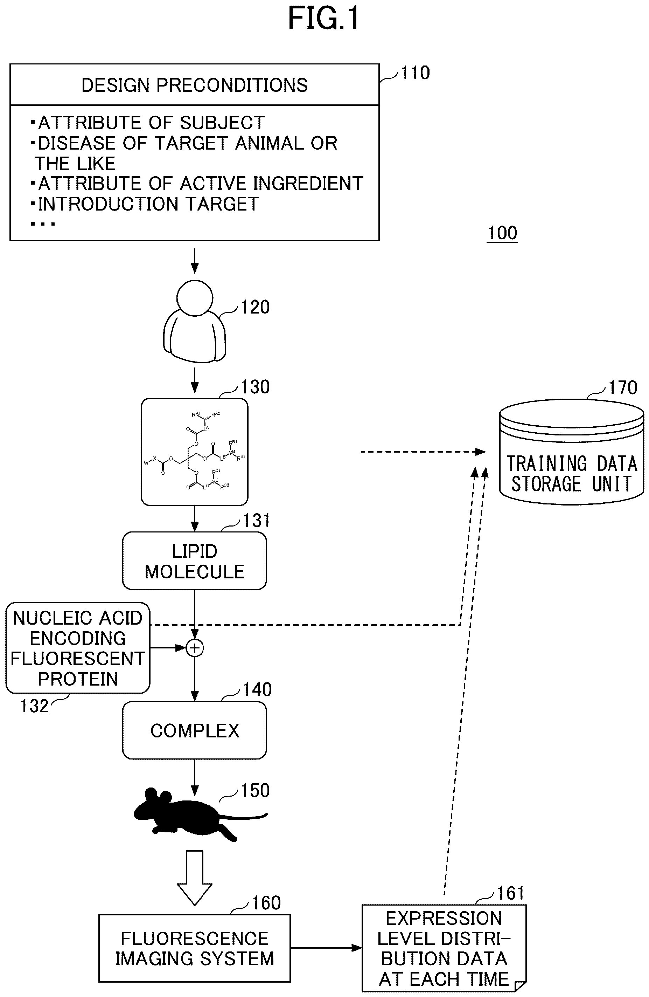

First, an example of collecting "expression level distribution data at each time", which is collected as training data in an evaluation system according to a first embodiment, will be described. FIG. 1 is a diagram illustrating an example of collecting the expression level distribution data.

In an evaluation system 100, when a chemical structure of a lipid molecule forming a particle including an active ingredient is designed or selected, first, as a precondition for design or selection (hereinafter, simply referred to as a "design precondition"), the following items and the like (see design preconditions 110) are input to a designer 120.
- Attribute of a subject (a human or an animal other than a human as a target (hereinafter, in the present specification, may be referred to as a "target animal or the like"));
- Type of a disease of the target animal or the like;
- Attribute of the encapsulated active ingredient (e.g., a nucleic acid) (e.g., a type, chemical structure information, and the like on the active ingredient (e.g., the nucleic acid));
- Target into which a particle of a complex 140 is introduced (a specific cell in a living organism (in vivo) of a target animal or the like 150).
Here, the particle encapsulating the active ingredient at least indicates a concept including:
- a case in which lipid molecules are mixed together with an active ingredient (e.g., the nucleic acid) to form a complex particle; or
- a case in which lipid molecules form a shell and an active ingredient (e.g., the nucleic acid) is included in the shell to form a complex particle.

Examples of lipid molecules handled in one aspect of the present disclosure include cationic lipid molecules. A cationic lipid indicates a lipid that has a net positive charge at a selected pH, such as physiological pH. Examples of a method for producing lipid molecules and a particle encapsulating an active ingredient include methods described in WO 2016/021683, WO 2019/131839, WO 2020/032184, and the like. Examples of lipid molecules handled in another aspect of the present disclosure include anionic lipid molecules, cholesterol derivative molecules, and amphiphilic lipid molecules.

In the present disclosure, the "active ingredient" refers to a substance having biological or pharmacological activity, and particularly refers to a substance useful for medicinal use or research purpose use (hereinafter, may be referred to as a "drug" in the present specification). Examples of the active ingredient include nucleic acids.

The nucleic acid handled in one aspect of the present disclosure may be any molecule as long as it is a molecule obtained by polymerizing nucleotides and polymerizing molecules having a function equivalent to the nucleotides, and examples thereof include RNA, which is a polymer of ribonucleotides, DNA, which is a polymer of deoxyribonucleotides, a polymer in which ribonucleotides and deoxyribonucleotides are mixed, and a nucleotide polymer containing nucleotide analogs, and may include a nucleotide polymer containing a nucleic acid derivative. Additionally, the nucleic acid may also be a single-stranded nucleic acid or a double-stranded nucleic acid. Additionally, the double-stranded nucleic acid includes a double-stranded nucleic acid in which one strand is hybridized by the other strand under a stringent condition. The nucleic acid handled in the present embodiment is not particularly limited, and may be, for example, a nucleic acid for the purpose of improving a disease, symptom, disorder, or pathological condition, alleviating a disease, symptom, disorder or pathological condition, or preventing the onset thereof (hereinafter, may be referred to as "treatment of a disease or the like" in the present specification), or a nucleic acid for regulating the expression of a desired protein useful for research although not contributing to the treatment of a disease or the like. Specific examples of nucleic acids handled in the present embodiment include siRNA, miRNA, miRNA mimic, antisense nucleic acids, ribozymes, mRNA, decoy nucleic acids, aptamers, DNA, and artificially modified analogs or derivatives thereof.

Here, a method of administering the complex handled in one aspect of the present disclosure includes in-vivo, ex-vivo, and in-situ administration methods and the like.

The designer 120 designs or selects the chemical structure of the lipid molecule from the design preconditions 110 based on the past experience and know-how, and determines lipid molecule chemical structure information 130.

When the lipid molecule chemical structure information 130 is determined by the designer 120, a lipid molecule 131 is generated based on the chemical structure information 130. Then, a nucleic acid 132 (RNA, DNA, or the like) encoding a fluorescent protein is encapsulated by the particle containing the lipid molecule 131, and the particle of the complex 140 is formed. Here, the particle of the complex 140 may contain components other than the lipid molecule 131 and the nucleic acid 132 encoding a fluorescent protein in addition to the nucleic acid 132 encoding a fluorescent protein, as necessary. Examples of such components include appropriate amounts of stabilizers and antioxidants. These components may be pharmaceutically acceptable components.

The formed particle of the complex 140 is administered to the target animal or the like 150.

A change caused by introducing the particle of the complex 140 into a specific cell in a living organism (in vivo) of the target animal or the like 150 is captured by a fluorescence imaging system 160.

The fluorescence imaging system 160 is a device that can acquire information indicating distribution or behavior of the active ingredient in the target animal or the like 150. Specifically, the fluorescence imaging system 160 is a device that captures the expression level of the fluorescent protein at each time and at each position in the target animal or the like 150 as fluorescence intensity distribution data at each time. As illustrated in FIG. 1, when the particle of the complex 140 is administered to the target animal or the like 150, the fluorescence imaging system 160 captures the fluorescence intensity distribution data indicating the expression level of the fluorescent protein at each time and at each position in the target animal or the like 150 at predetermined time intervals. Additionally, the fluorescence imaging system 160 outputs the captured fluorescence intensity distribution data at each time as expression level distribution data 161 at each time.

The expression level distribution data 161 at each time that is output by the fluorescence imaging system 160 is stored in a training data storage unit 170 as training data together with the chemical structure information 130 on the corresponding lipid molecule and the type of the corresponding active ingredient.

Here, the above method of collecting the expression level distribution data is an example, and another collection method may be used. For example, the expression level distribution data can also be acquired by administering the particle of the complex 140 into the living organism of the target animal or the like 150, then extracting each tissue or cell from the living organism at a certain time, and measuring the expression level in each tissue or cell.

### <Example of Collecting PK Data and PD Data>

Next, an example of collecting "PK data and PD data", which are collected as the training data in the evaluation system according to the first embodiment, will be described.

In the present disclosure, the PK data indicates Pharmacokinetics (PK) data and PD data indicates Pharmacodynamics (PD) data. The PK data represents a relationship between administration and dosage of a drug; and a change in the concentration of the drug in the living organism (absorption, distribution, metabolism, and excretion). The PD data represents a relationship between exposure and effect (expected effect and side effect) of a drug in the living organism. In the present disclosure, PK/PD indicates a relationship between PK and PD. For example, a method of quantitatively evaluating a change in a biomarker by a model formula depending on a drug concentration is known. In the field of antibacterial drugs, a relationship between a composite parameter (e.g., Cmax/MIC) and a therapeutic effect is studied. The composite parameter uses the minimum inhibitory concentration (MIC) as an index of PD and the drug maximum blood concentration (Cmax) as an index of PK. As described above, the type of the PK data, the type of the PD data, and the analysis method used in the PK/PD analysis and the used data thereof can be appropriately selected by a person skilled in the art.

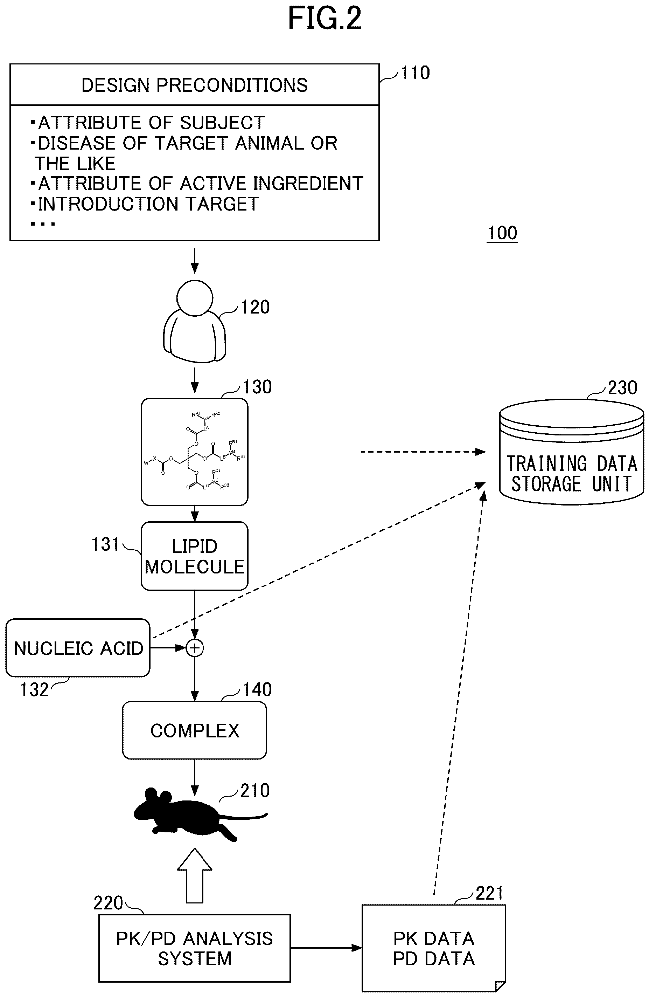

FIG. 2 is a diagram illustrating an example of collecting the PK data and the PD data. A difference from FIG. 1 is that the particle of the complex 140 is formed by a particle containing the lipid molecule 131 encapsulating a nucleic acid 133 instead of or in addition to the nucleic acid 132 encoding the fluorescent protein. Additionally, the difference from FIG. 1 is that when the formed particle of the complex 140 is administered to the target animal or the like 150, measurements are performed by a PK/PD analysis system 220. In the present disclosure, as the nucleic acid 133, a nucleic acid for the purpose of improving a disease, a symptom, a disorder, or a pathological condition and alleviating a disease, a symptom, a disorder, or a pathological condition, or preventing the onset thereof (in the present specification, may be referred to as "treatment of a disease or the like") can be used.

The PK/PD analysis system 220 is a device that acquires information indicating the distribution or behavior of the active ingredient in the target animal or the like 150. Specifically, the PK/PD analysis system 220 measures and outputs:
- a change over time in any one or more of a tissue concentration, a blood concentration, or a urine concentration of the active ingredient; and
- a relationship between any one or more of a tissue concentration, a blood concentration, or a urine concentration of the active ingredient; and a pharmacological effect and/or toxicity,
as PK data and PD data 221.

The PK data and the PD data 221 output by the PK/PD analysis system 220 are stored in the training data storage unit 230 as the training data together with the chemical structure information on the corresponding active ingredient and the type of the corresponding active ingredient.

Here, the type and value of the target pharmacological effect and/or toxicity may be appropriately selected by a person skilled in the art based on the type of a target disease, the type of a target tissue and cell, the expected therapeutic effect, the type of the expected toxicity, and the like.

Examples of the type of the target toxicity include the production amount or the concentration in tissue or blood of various cytokines and chemokines (e.g., inflammatory cytokines (e.g., tumor necrosis factor (TNF), interleukin (IL)-1, IL-6, IL-8, IL-12, IL-18, and the like)), the body weight, a histopathological state, an appearance, a behavior, activity amount, hepatotoxicity evaluation index (e.g., alanine aminotransferase (ALT), aspartate aminotransferase (AST)), hepatobiliary disorder evaluation index (e.g., alkaline phosphatase (ALP), gamma-glutamyltransferase (GGT), total bilirubin (T.BIL)), and changes over time thereof.

### <Outline of Process in Evaluation System>

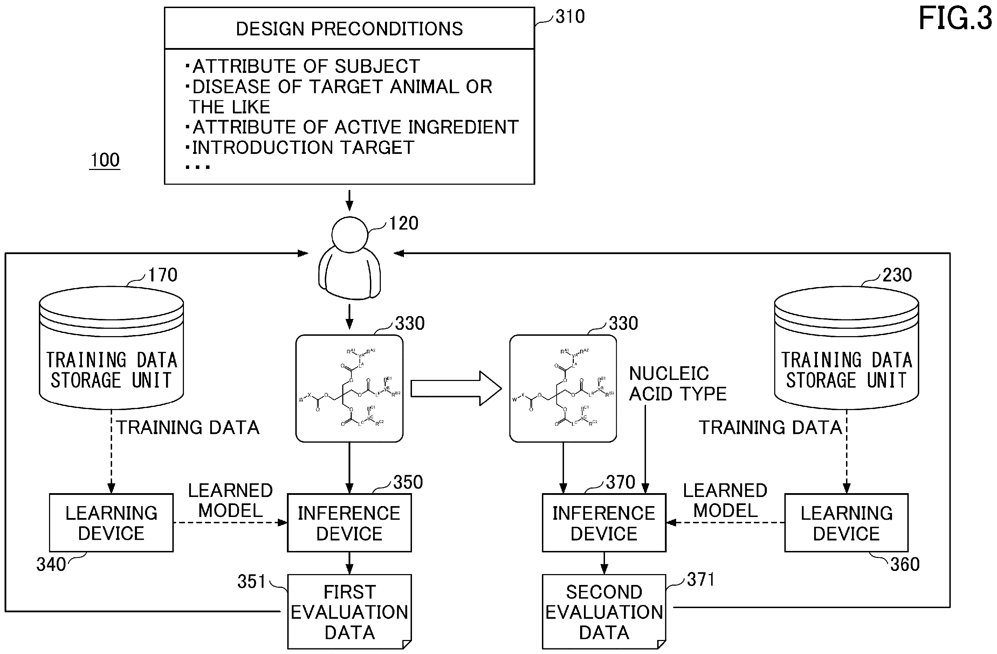

Next, an outline of a process in the evaluation system 100 will be described. FIG. 3 is a first diagram for explaining the outline of the process in the evaluation system. As illustrated in FIG. 3, the evaluation system 100 includes a learning device 340, an inference device 350, a learning device 360, and an inference device 370.

The learning device 340 reads the training data stored in the training data storage unit 170, and learns a relationship between the type of the active ingredient and the chemical structure information on the lipid molecule; and the expression level distribution data at each time. Here, a learned model (model parameters of the learned model) in which the relationship between the type of the active ingredient and the chemical structure information on the lipid molecule; and the expression level distribution data has been learned by the learning device 340 is set in the inference device 350.

The inference device 350 outputs expression level data of a designated organ at each time in response to chemical structure information 330 on a lipid molecule newly designed or selected by the designer 120 and the type of the active ingredient being input. The expression level data of the designated organ at each time output by the inference device 350 is notified to the designer 120 as first evaluation data 351.

The learning device 360 reads the training data stored in the training data storage unit 230, and learns a relationship between the type of the active ingredient and the chemical structure information on the lipid molecule; and the PK data and the PD data. Here, a learned model (model parameters of the learned model) in which the relationship between the type of the active ingredient and the chemical structure information on the lipid molecule; and the PK data and the PD data has been learned by the learning device 360 is set in the inference device 370.

The inference device 370 outputs a feature of the PK data, a feature of the PD data, and a feature of the PK/PD data in response to the chemical structure information 330 on the lipid molecule newly designed or selected by the designer 120 and the type of the active ingredient being input. The feature of the PK data, the feature of the PD data, and the feature of the PK/PD data output by the inference device 370 are notified to the designer 120 as second evaluation data 371.

As described above, each time the designer 120 newly designs or selects the chemical structure of the lipid molecule and designates the chemical structure information 330, the inference devices 350 and 370 notify the designer 120 of the first evaluation data 351 and the second evaluation data 371.

This allows the designer 120 to design or select the chemical structure of the new lipid molecule while referring to the first evaluation data 351 and the second evaluation data 371.

### <Hardware Configurations of Learning Device and Inference Device>

Next, hardware configurations of the learning device 340, the inference device 350, the learning device 360, and the inference device 370 will be described. Here, the learning device 340 to the inference device 370 have substantially the same hardware configurations, and thus the hardware configuration of the inference device 350 will be described here.

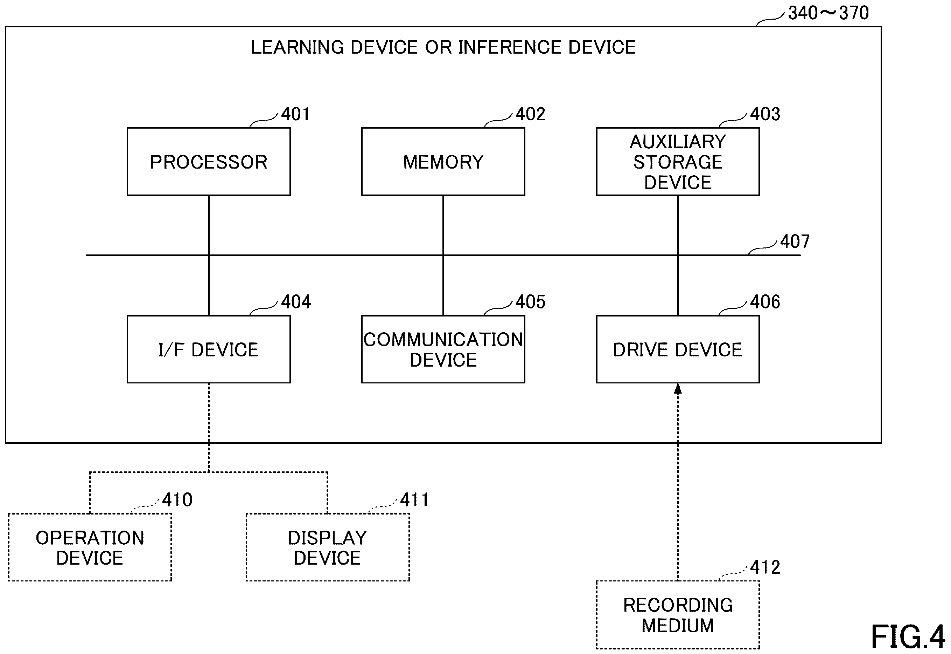

FIG. 4 is a diagram illustrating an example of the hardware configuration of the inference device. As illustrated in FIG. 4, the inference device 350 includes a processor 401, a memory 402, an auxiliary storage device 403, an interface (I/F) device 404, a communication device 405, and a drive device 406. Here, the hardware components of the inference device 350 are connected to each other via a bus 407.

The processor 401 includes various computing devices such as a central processing unit (CPU), a graphics processing unit (GPU), or the like. The processor 401 reads various programs installed in the auxiliary storage device 403 onto the memory 402 and executes the programs.

The memory 402 includes a main storage device such as a read only memory (ROM), a random access memory (RAM), or the like. The processor 401 and the memory 402 form what is called a computer, and the processor 401 executes various programs read on the memory 402, and the computer realizes various functions.

The auxiliary storage device 403 stores various programs and various data used when the various programs are executed by the processor 401.

The I/F device 404 is a connection device that connects an operation device 410 and a display device 411 to the inference device 350. The I/F device 404 receives various instructions for the inference device 350 via the operation device 410. Additionally, the I/F device 404 outputs a processing result by the inference device 350 via the display device 411.

The communication device 405 is a communication device for communicating with another device via a network.

The drive device 406 is a device for setting a recording medium 412. The recording medium 412 here includes a medium for optically, electrically, or magnetically recording information, such as a CD-ROM, a flexible disk, a magneto-optical disk, or the like. Additionally, the recording medium 412 may include a semiconductor memory or the like that electrically records information, such as a ROM, a flash memory, or the like.

Here, the various programs to be installed in the auxiliary storage device 403 are installed by, for example, the distributed recording medium 412 being set in the drive device 406 and the various programs recorded in the recording medium 412 being read by the drive device 406. Alternatively, the various programs to be installed in the auxiliary storage device 403 may be installed by being downloaded from a network via the communication device 405.

### <Functional Configuration of Learning Device>

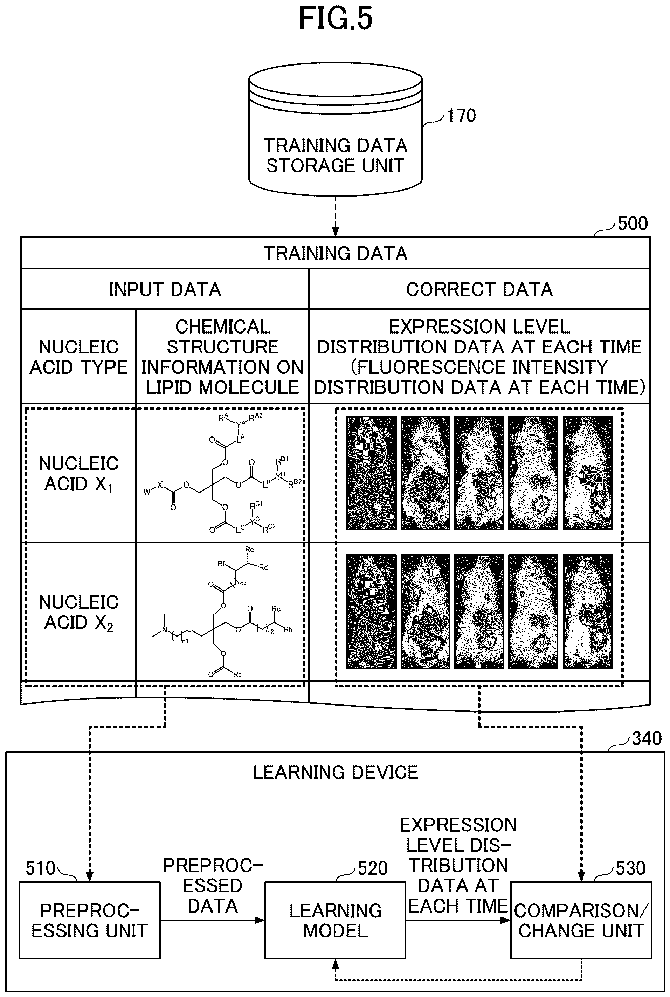

Next, a functional configuration of the learning device 340 will be described. FIG. 5 is a first diagram illustrating an example of the functional configuration of the learning device. In FIG. 5, training data 500 is an example of the training data generated based on the expression level distribution data 161 at each time stored in the training data storage unit 170. Here, although items of each information on the training data (input data and correct data) are exemplified below, the items are not limited thereto, and another item may be used as the training data, or some of these items may be used as the training data.

As illustrated in FIG. 5, the training data 500 includes input data and correct data, and the input data includes, as the items of the information, "nucleic acid type" and "chemical structure information on lipid molecule".

In "nucleic acid type", "nucleic acid X₁", "nucleic acid X₂", and the like are stored as the types of the nucleic acids corresponding to the target diseases. In "chemical structure information on lipid molecule", chemical structure information that determines the chemical structure of the lipid molecule designed or selected by the designer 120 in the past.

With respect to the above, the correct data includes, as the items of the information, "expression level distribution data at each time (fluorescence intensity distribution data at each time) ".

In "expression level distribution data at each time (fluorescence intensity distribution data at each time)", an image indicating an expression level expressed at each position in the living organism at each time, after the particle of the complex in which the corresponding nucleic acid (nucleic acid encoding a fluorescent protein) is encapsulated in the particle containing the corresponding lipid molecule is administered to the living organism of the target animal or the like 150. The example of FIG. 5 indicates a state in which expression level distribution data at five different times are stored, and a difference in color for each pixel in the respective expression level distribution data indicates that the expression level of the active ingredient differs in accordance with a position in the living organism, corresponding to the pixel. Additionally, the difference in the colored positions, colored ranges, and colors between the respective expression level distribution data indicates that the expression level, and the position and range of the expression in the living organism change as the time passes.

Here, these training data may be data obtained from various public information (for example, patent publications and papers) or databases.

With respect to the above, a learning program is installed in the learning device 340, and by the learning program being executed, the learning device 340 functions as a preprocessing unit 510, a learning model 520, and a comparison/change unit 530.

The preprocessing unit 510 acquires "input data" of the training data 500 and performs various preprocessing to generate preprocessed data suitable for being input into the learning model 520. The various preprocessing performed by the preprocessing unit 510 include processing for normalizing the input data, processing for vectorizing the input data, and the like.

The learning model 520 is a model that associates the input data (the type of the nucleic acid and the chemical structure information on the lipid molecule) with the correct data (the expression level distribution data at each time). Specifically, the learning model 520 receives, as input, the preprocessed data notified from the preprocessing unit 510, and outputs, as output data, the expression level distribution data at each time.

Here, on the learning model 520, a learning process of updating the model parameters by back-propagating the error calculated by the comparison/change unit 530 is performed. This generates a learned model. That is, the learned model is generated by updating the model parameters of the learning model 520 so that output data of the learning model 520 approaches the correct data (the expression level distribution data at each time).

The comparison/change unit 530 calculates the error by comparing expression level distribution data at each time, output from the learning model 520, with the correct data (the expression level distribution data at each time) of the training data 500. Additionally, the comparison/change unit 530 updates the model parameters of the learning model 520 by performing back propagation of the calculated error.

### <Functional Configuration of Inference Device>

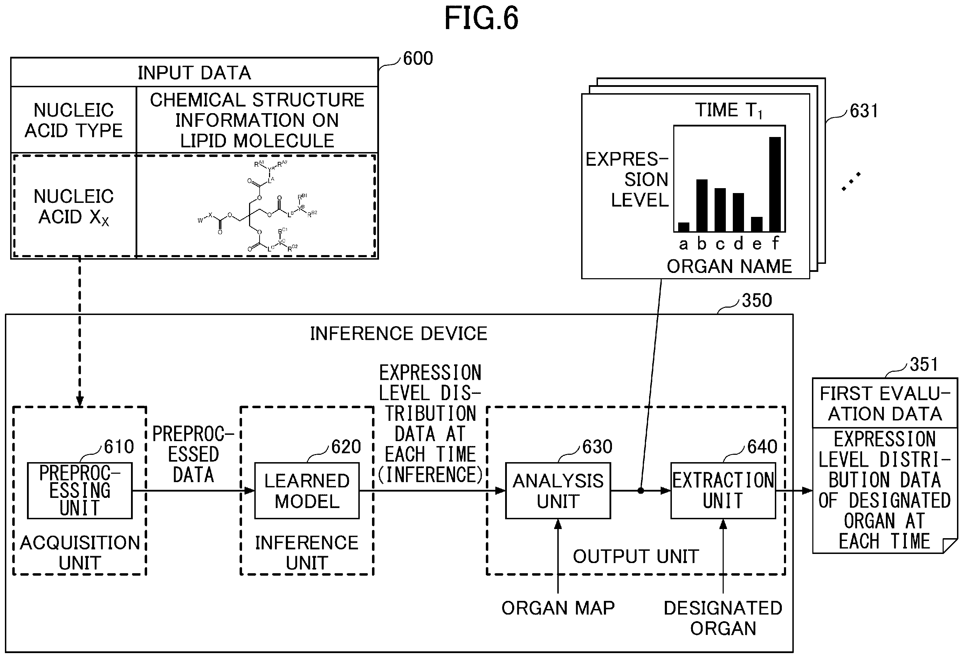

Next, a functional configuration of the inference device 350 will be described in detail. FIG. 6 is a first diagram illustrating an example of the functional configuration of the inference device. As illustrated in FIG. 6, input data 600 includes the type of the nucleic acid included in the design precondition 110 and chemical structure information determining the chemical structure of the lipid molecule designed or selected by the designer 120 based on the design precondition 110.

Here, the input data may be data obtained from various public information (for example, patent documents and papers) or databases.

An inference program is installed in the inference device 350, and by the inference program being executed, the inference device 350 functions as a preprocessing unit 610, a learned model 620, an analysis unit 630, and an extraction unit 640.

The preprocessing unit 610 is an example of an acquisition unit and has the same function as the preprocessing unit 510 included in the learning device 340. Specifically, the preprocessing unit 610 acquires the input data 600 and performs various preprocessing on the acquired input data 600 to generate preprocessed data.

The learned model 620 is an example of an inference unit, and is generated by the learning device 340 performing a learning process. In response to the preprocessed data notified from the preprocessing unit 610 being input, the learned model 620 infers expression level distribution data at each time.

The analysis unit 630 and the extraction unit 640 are examples of an output unit. Among these, the analysis unit 630 acquires an organ map for determining which position of the tissue and/or cell (here, the organ) in the living organism of the target animal corresponds to each position in the expression level distribution data at each time. Additionally, the analysis unit 630 calculates an expression level of each organ based on the expression level distribution data at each time, inferred by the learned model 620, and the organ map. In FIG. 6, the horizontal axis of the reference numeral 631 represents the organ name, and the vertical axis represents the expression level. That is, the reference numeral 631 in FIG. 6 represents the expression level of each organ at each time.

The extraction unit 640 outputs the expression level in the designated organ. Specifically, the extraction unit 640 extracts the expression level of the designated organ from among the expression levels of the respective organs calculated by the analysis unit 630. Additionally, the extraction unit 640 notifies the designer 120 of the extracted expression level data of the designated organ at each time (a change over time in the expression level in the designated organ) as the first evaluation data 351. That is, the first evaluation data 351 is information indicating the distribution or behavior of the specific tissue and/or cell in the target animal or the like 150. Here, the extraction unit 640 may notify the designer 120 of the first evaluation data 351 after interpolating the expression level data between the times by using the extracted expression level data of the designated organ at each time.

### <Functional Configuration of Learning Device>

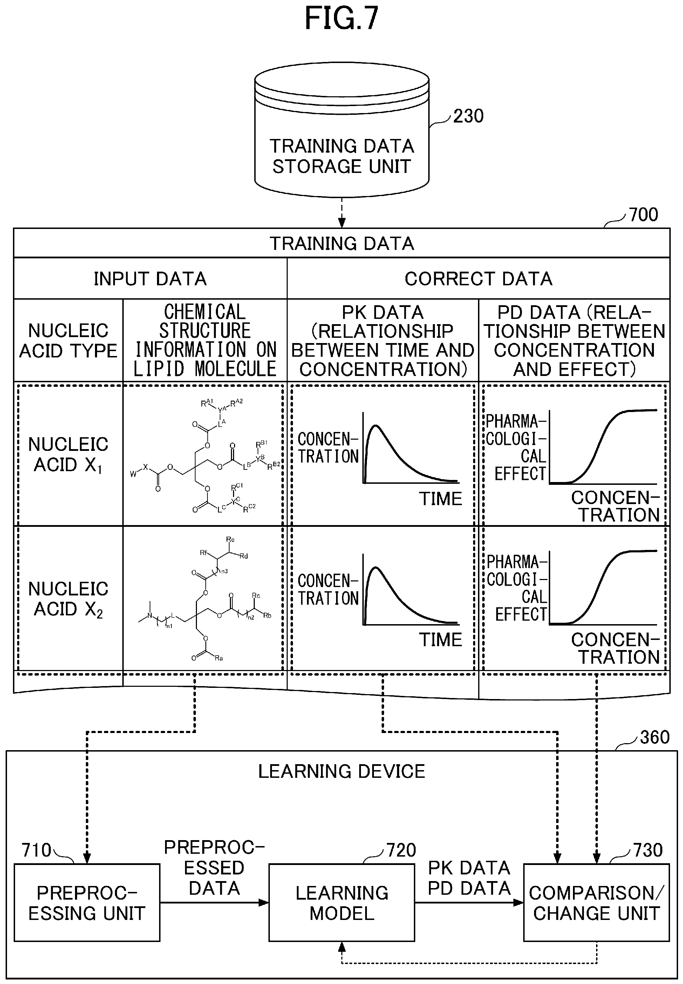

Next, a functional configuration of the learning device 360 will be described. FIG. 7 is a second diagram illustrating an example of the functional configuration of the learning device. In FIG. 7, training data 700 is an example of training data generated based on the PK data and the PD data 221 stored in the training data storage unit 230. Here, although items of each information of the training data (input data and correct data) are exemplified below, the items are not limited thereto, and another item may be used as the training data, or some of these items may be used as the training data.

As illustrated in FIG. 7, the training data 700 includes input data and correct data, and the input data includes, as the items of the information, "nucleic acid type" and "chemical structure information on lipid molecule".

In "nucleic acid type", "nucleic acid X₁", "nucleic acid X₂", and the like are stored as the types of the nucleic acids corresponding to the target diseases. In "chemical structure information on lipid molecule", chemical structure information that determines the chemical structure of the lipid molecule designed or selected by the designer 120 in the past is stored.

With respect to the above, the correct data includes, as the items of the information, "PK data" and "PD data". "PK data" stores information indicating any one or more of a tissue concentration, a blood concentration, or a urine concentration of the nucleic acid at each time when the particle of the complex in which the corresponding nucleic acid is encapsulated in the particle containing the corresponding lipid molecule is administered to the target animal or the like 150.

"PD data" stores information indicating the relationship between any one or more of a tissue concentration, a blood concentration, or a urine concentration of the nucleic acid; and the pharmacological effect and/or toxicity of the nucleic acid when the particle of the complex in which the corresponding nucleic acid is encapsulated in the particle containing the corresponding lipid molecule is administered to the target animal or the like 150.

Here, these training data may be data obtained from various public information (for example, patent publications and papers) or databases.

A learning program is installed in the learning device 360, and by the learning program being executed, the learning device 360 functions as a preprocessing unit 710, a learning model 720, and a comparison/change unit 730.

The preprocessing unit 710 acquires "input data" of the training data 700 and performs various preprocessing to generate preprocessed data suitable for being input into the learning model 720. The various preprocessing performed by the preprocessing unit 710 include processing for normalizing the input data, processing for vectorizing the input data, and the like.

The learning model 720 is a model that associates the input data (the type of the nucleic acid and the chemical structure information on the lipid molecule) with the correct data (the PK data and the PD data). Specifically, the learning model 720 receives, as input, the preprocessed data notified from the preprocessing unit 710, and outputs, as output data, the PK data and the PD data.

Here, on the learning model 720, a learning process of updating the model parameters by back-propagating the error calculated by the comparison/change unit 730 is performed. This generates a learned model. That is, the learned model is generated by updating the model parameters of the learning model 720 so that output data of the learning model 720 approaches the correct data (the PK data and the PD data).

The comparison/change unit 730 calculates the error by comparing the PK data and the PD data output from the learning model 720 with the correct data (the PK data and the PD data) of the training data 700. Additionally, the comparison/change unit 730 updates the model parameters of the learning model 720 by back-propagating the calculated error.

### <Functional Configuration of Inference Device>

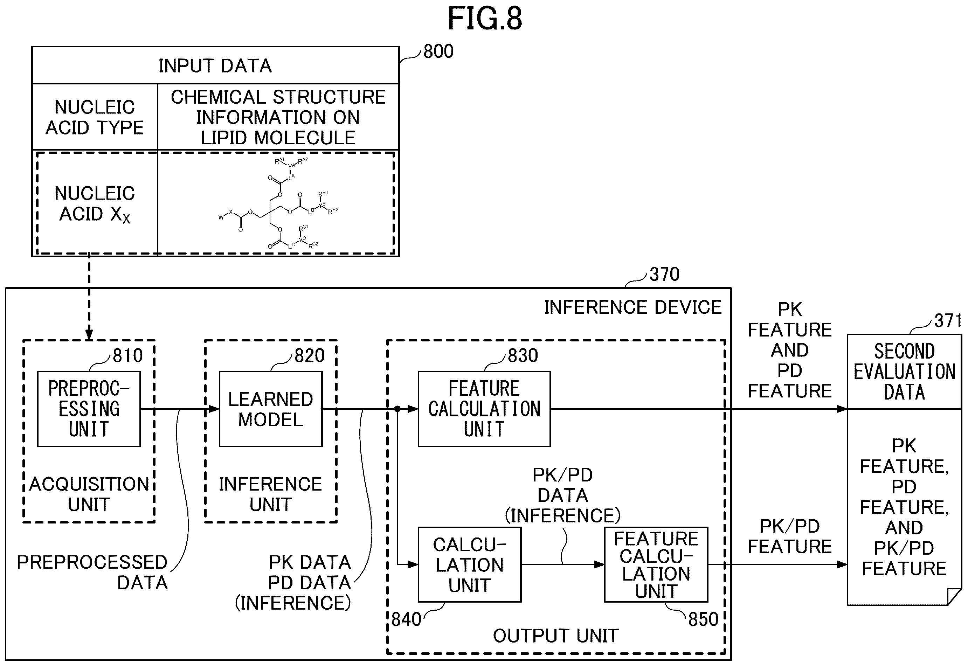

Next, a functional configuration of the inference device 370 will be described in detail. FIG. 8 is a second diagram illustrating an example of the functional configuration of the inference device. As illustrated in FIG. 8, input data 800 includes the type of the nucleic acid included in the design precondition 110 and the chemical structure information determining the chemical structure of the lipid molecule designed or selected by the designer 120 based on the design precondition 110.

Here, the input data may be data obtained from various public information (for example, patent documents and papers) or databases.

An inference program is installed in the inference device 370, and by the inference program being executed, the inference device 370 functions as a preprocessing unit 810, a learned model 820, a feature calculation unit 830, a calculation unit 840, and a feature calculation unit 850.

The preprocessing unit 810 is an example of the acquisition unit and has the same function as the preprocessing unit 710 included in the learning device 360. Specifically, the preprocessing unit 810 acquires the input data 800 and performs various preprocessing on the acquired input data 800 to generate preprocessed data.

The learned model 820 is an example of the inference unit, and is generated by the learning device 360 performing a learning process. In response to the preprocessed data notified from the preprocessing unit 810 being input, the learned model 820 infers PK data and PD data.

The feature calculation unit 830, the calculation unit 840, and the feature calculation unit 850 are an example of the output unit. Among these, the feature calculation unit 830 calculates, as the PK feature, the feature of the PK data inferred by the learned model 820. Additionally, the feature calculation unit 830 calculates, as the PD feature, the feature of the PD data inferred by the learned model 820.

The PK feature and the PD feature include:
- a feature related to a change over time in any one or more of a tissue concentration, a blood concentration, or a urine concentration of the nucleic acid; and
- a feature indicating a relationship between any one or more of a tissue concentration, a blood concentration, or a urine concentration; and a pharmacological effect and/or toxicity.

Specifically, the PK feature may include:
- a maximum value of any one of a tissue concentration, a blood concentration, and a urine concentration of the nucleic acid or any multiple maximum values thereof;
- a time length to reach the maximum value;
- a time length from when any one or more of a tissue concentration, a blood concentration, or a urine concentration of the nucleic acid reaches the maximum value to when the concentration decreases to a predetermined reference value or less; and
- any one or more of the tissue concentration, the blood concentration, or the urine concentration of the nucleic acid at a predetermined time.

Similarly, the PD feature includes:
- any one or more of a tissue concentration, a blood concentration, or a urine concentration of the nucleic acid at which the pharmacological effect and/or toxicity becomes constant;
- the median effect concentration (EC50);
- a value of the pharmacological effect and/or toxicity at that time; and
- any one or more of a tissue concentration, a blood concentration, or a urine concentration of the nucleic acid at which the pharmacological effect and/or toxicity significantly changes.

The calculation unit 840 calculates the PK/PD data based on the PK data and the PD data inferred by the learned model 820. Additionally, the calculated PK/PD data is notified to the feature calculation unit 850.

The feature calculation unit 850 calculates, as the PK/PD feature, the feature of the PK/PD data calculated by the calculation unit 840.

Here, the PK/PD feature calculated by the feature calculation unit 850 includes any suitable feature. For example, the PK/PD feature may include the maximum value of the pharmacological effect and/or toxicity, or a time length to reach the maximum. Alternatively, the PK/PD feature may include a time length from when the pharmacological effect and/or toxicity reaches the maximum value to when the pharmacological effect and/or toxicity decreases to a predetermined reference value or less, or the pharmacological effect and/or toxicity at a predetermined time.

The PK feature and the PD feature calculated by the feature calculation unit 830 and the PK/PD feature calculated by the feature calculation unit 850 are notified to the designer 120 as the second evaluation data 371. That is, the second evaluation data 371 is information indicating the behavior in any one or more of the tissue, blood, or urine in the target animal or the like 150.

### <Evaluation Process>

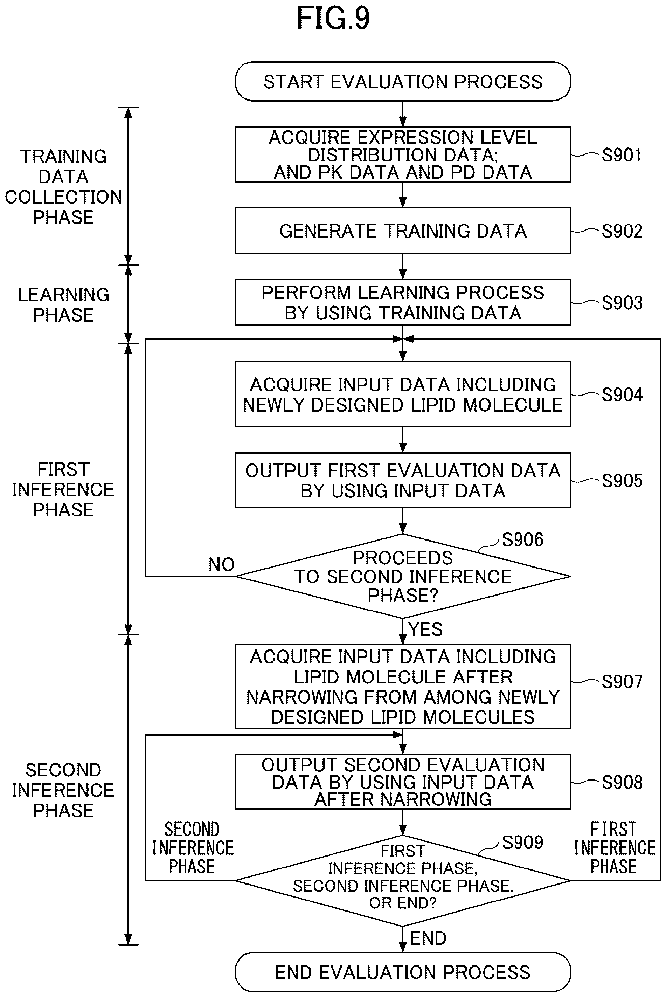

Next, a flow of an evaluation process in the evaluation system 100 will be described. FIG. 9 is a first flowchart illustrating the flow of the evaluation process. As illustrated in FIG. 9, the evaluation process includes a training data collection phase, a learning phase, a first inference phase, and a second inference phase.

First, when the process proceeds to the training data collection phase, in step S901, the learning device 340 and the learning device 360 respectively acquire the expression level distribution data 161 at each time; and the PK data and the PD data 221.

In step S902, the learning device 340 and the learning device 360 respectively generate the training data 500 and the training data 700.

Subsequently, when the process proceeds to the learning phase, in step S903, the learning device 340 performs a learning process by using the training data 500 to generate the learned model 620. Additionally, the learning device 360 performs a learning process by using the training data 700 to generate the learned model 820.

Subsequently, when the process proceeds to the first inference phase, in step S904, the inference device 350 acquires the input information including the chemical structural information determining the chemical structure of the lipid molecule newly designed or selected by the designer 120.

In step S905, the inference device 350 inputs the acquired input data into the learned model 620 to output the first evaluation data.

In step S906, the designer 120 determines whether to proceed to the second inference phase. If the first evaluation data does not satisfy a predetermined condition, the designer 120 determines not to proceed to the second inference phase (NO in step S906), and the process returns to step S904.

If the first evaluation data satisfies the predetermined condition, the designer 120 determines to proceed to the second inference phase (YES in step S906), and the process proceeds to step S907.

Subsequently, the process proceeds to the second inference phase. In step S907, the inference device 370 acquires, from among the chemical structure information determining the chemical structures of multiple lipid molecules newly designed or selected by the designer 120, the input data including the chemical structure of the lipid molecule that is determined to satisfy the predetermined condition. That is, the inference device 370 acquires the input data after the narrowing.

In step S908, the inference device 370 inputs the acquired input data into the learned model 820 to output the second evaluation data.

In step S909, the designer 120 determines whether to return to the first inference phase, continue the second inference phase, or end the second inference phase.

If all the input data acquired in step S907 are input into the learned model 820 and it is determined that none of the second evaluation data satisfies the predetermined condition, the process returns to step S904 of the first inference phase.

If it is determined that there is input that has not been input into the learned model 820 among the input data acquired in step S907, the process returns to step S908 of the second inference phase.

If all the input data acquired in step S907 are input into the learned model 820 and it is determined that any of the second evaluation data satisfies the predetermined condition, the second inference phase is ended. In this case, the chemical structure of the lipid molecule included in the input data determined to satisfy the predetermined condition is the chemical structure of the lipid molecule searched by the designer 120.

### <Summary>

As is clear from the above description, the evaluation system 100 according to the first embodiment is configured as follows.
- The preprocessing unit 610 or the preprocessing unit 810 acquires the input data including at least the chemical structure information on the lipid molecule.
- The learned model 620 that associates the input data including at least the chemical structure information on the lipid molecule with the information (the expression level distribution data at each time) indicating the distribution or behavior of the active ingredient encapsulated in the particle containing the lipid molecule in the living organism is generated. Alternatively, the learned model 820 that associates the input data including at least the chemical structure information on the lipid molecule with the information (the PK data and the PD data) indicating the distribution or behavior of the active ingredient encapsulated in the particle containing the lipid molecule in the living organism is generated.

- The input data newly acquired by the preprocessing unit 610 is input into the learned model 620, and the information (the expression level distribution data at each time) indicating the distribution or behavior in the living organism, associated with the newly acquired input data, is inferred. Alternatively, the input data newly acquired by the preprocessing unit 810 is input into the learned model 820, and the information (the PK data and the PD data) indicating the distribution or behavior in the living organism, associated with the newly acquired input data, is inferred.
- The information (the change over time in the expression level in the designated organ) indicating the distribution or behavior in the predetermined tissue and/or cell is output based on the inferred information (the expression level distribution data at each time) indicating the distribution or behavior in the living organism.
- The information (the PK feature, the PD feature, and the PK/PD feature) indicating the behavior in any one or more of the predetermined tissue, blood, or urine is output based on the inferred information (the PK data and the PD data) indicating the distribution or behavior in the living organism.

With this, according to the first embodiment, each time the designer 120 newly designs or selects the chemical structure of the lipid molecule, the designer 120 can acquire, without performing experiments, the following:
- the information indicating the distribution or behavior of the active ingredient encapsulated in the lipid molecule in the predetermined tissue and/or cell in the living organism; or
- the information indicating the behavior of the active ingredient encapsulated in the lipid molecule in any one or more of the tissue, blood, or urine. That is, according to the evaluation system 100 of the first embodiment, the operation of designing or selecting the chemical structure of the lipid molecule forming the particle encapsulating the active ingredient can be supported.

### <Example 1>

Next, an example where the evaluation system 100 according to the first embodiment is used will be described. Here, an example where, among the learning devices 340 and 360 and the inference devices 350 and 370 included in the evaluation system 100, the learning device 340 is operated to generate the learned model, and the inference device 350 is operated to infer the expression level distribution data will be described.

### (1) Training Data and Verification Data

When the learning device 340 was operated, a data set that includes, as the input data, "chemical structure information on lipid molecule", "volume of active molecules", "buffer composition in particle", and "particle diameter", and that includes, as the correct data, "expression level distribution data" (a measured value) corresponding to each input data was prepared. Additionally, the prepared data set was divided into training data and verification data.

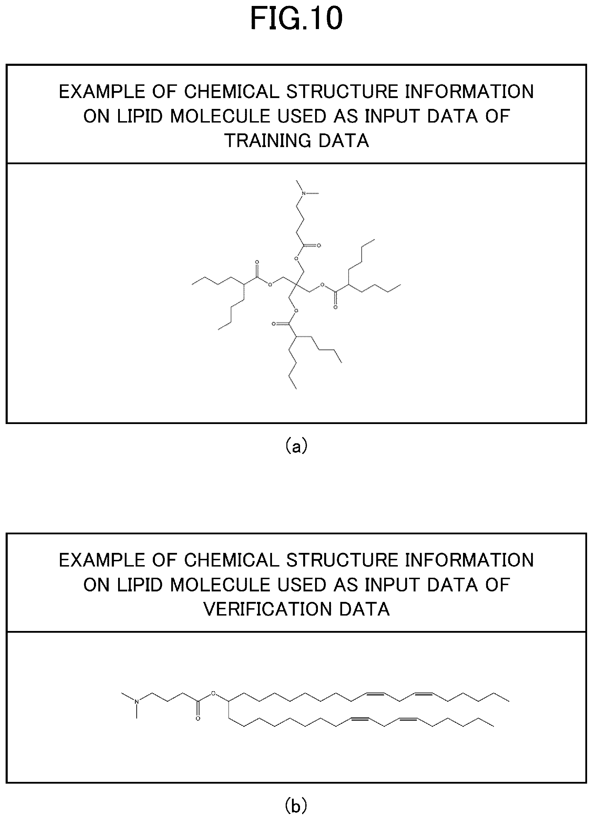

Subsequently, the learning device 340 was operated to perform a learning process by using the training data to generate the learned model 620. Additionally, the inference device 350 was operated to perform an inference process by using the verification data to verify the inference accuracy of the learned model 620. FIG. 10 is a first diagram illustrating a specific example of the training data and the verification data. Among these, FIG. 10 (a) illustrates an example of "chemical structure information on lipid molecule" used as the input data of the training data. FIG. 10 (b) illustrates an example of "chemical structure information on lipid molecule" used as the input data of the verification data.

### (2) Procedure for Generating Learned Model

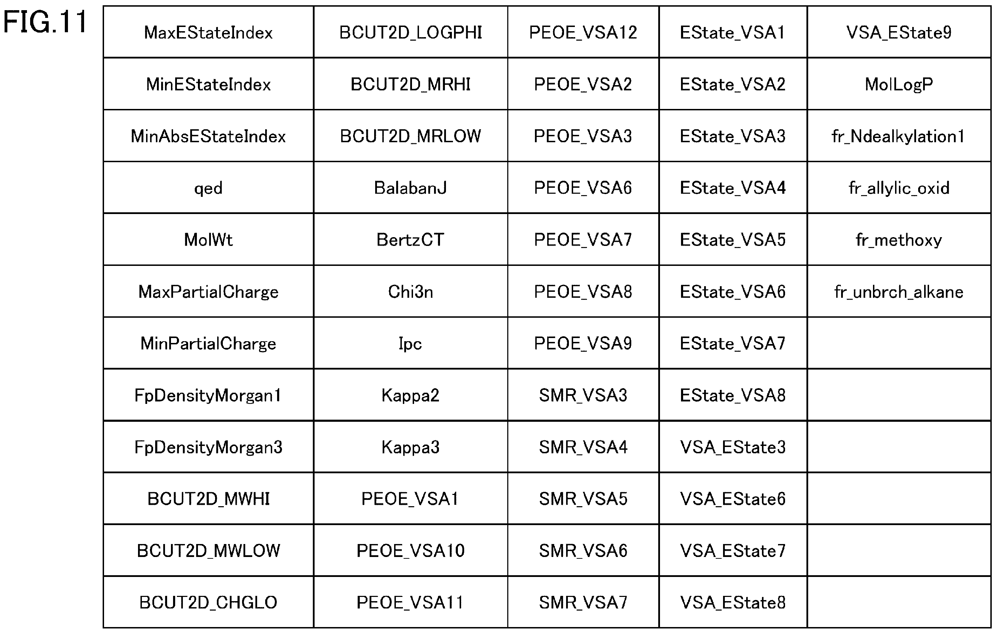

The learning process of generating the learned model 620 by using the training data was performed in the following procedure.
- The chemical structure information on the lipid molecule is converted into 208 molecular descriptors, and are combined with information on the volume of the active molecules, the buffer composition in the particle, and the particle diameter. Here, FIG. 11 is a first diagram illustrating a specific example of the molecular descriptor.
- At this time, molecular descriptors having small variances are deleted from the 208 molecular descriptors.
- Also, molecular descriptors that are found to be collinear are deleted.
- The learning process is performed by inputting the information on the volume of the active molecules, the buffer composition in the particle, and the particle diameter, which are combined with the molecular descriptor that is not deleted, as the input data, and the expression level distribution data (the measured value), as correct data, into the learning model. As the learning model, one-versus-the-rest random forest classification is used.

### (3) Procedure for Verifying Inference Accuracy of Learned Model

The verification process of verifying the inference accuracy of the learned model 620 by using the verification data was performed in the following procedure.
- The chemical structure information on the lipid molecule is converted into the molecular descriptor used when the learned model 620 is generated, and is combined with the information on the volume of the active molecules, the buffer composition in the particle, and the particle diameter.
- The information on the volume of the active molecules, the buffer composition in the particle, and the particle diameter, which are combined with the molecular descriptor, is input into the learned model 620, and the expression level distribution data is inferred.
- The inferred expression level distribution data and the measured expression level distribution data are compared to verify the inference accuracy by using the receiver operating characteristic (ROC) curve and the area under the curve (AUC) of each organ as the indices.

### (4) Verification Result

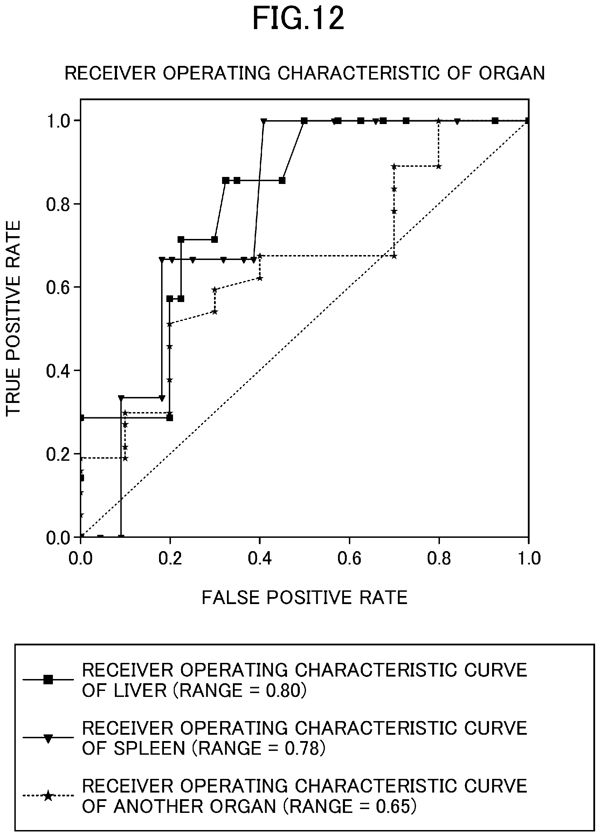

The inference accuracy of the learned model 620 was verified with respect to 47 types of lipid molecules, and the results are as illustrated in FIG. 12. FIG. 12 is a first diagram indicating a specific example of the verification result. As indicated in FIG. 12, it has been verified that the expression level distribution data inferred with respect to 47 types of lipid molecules generally have good inference accuracy.

### <Example 2>

Next, another example where the evaluation system 100 according to the first embodiment is used will be described. Here, an example where, among the learning devices 340 and 360 and the inference devices 350 and 370 included in the evaluation system 100, the learning device 360 is operated to generate the learned model and the inference device 370 is operated to infer the PK data will be described.

### (1) Training Data and Verification Data

When the learning device 360 was operated, a data set that includes "chemical structure information on lipid molecule" as the input data and that includes "PK data" (measured value) corresponding to the input data as correct data was prepared. Additionally, the prepared data set was divided into training data and verification data.

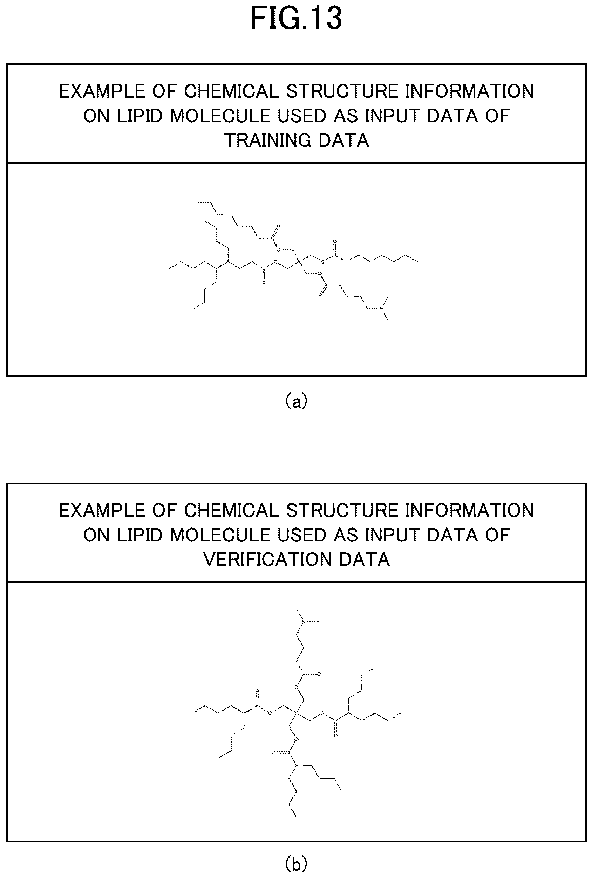

Subsequently, the learning device 360 was operated to perform a learning process by using the training data to generate the learned model 820. Additionally, the inference device 370 was operated to perform an inference process by using the verification data to verify the inference accuracy of the learned model 820. FIG. 13 is a second diagram illustrating a specific example of the training data and the verification data. Among these, FIG. 13 (a) illustrates an example of "chemical structure information on lipid molecule" used as the input data of the training data. FIG. 13 (b) illustrates an example of "chemical structure information on lipid molecule" used as the input data of the verification data.

### (2) Procedure for Generating Learned Model

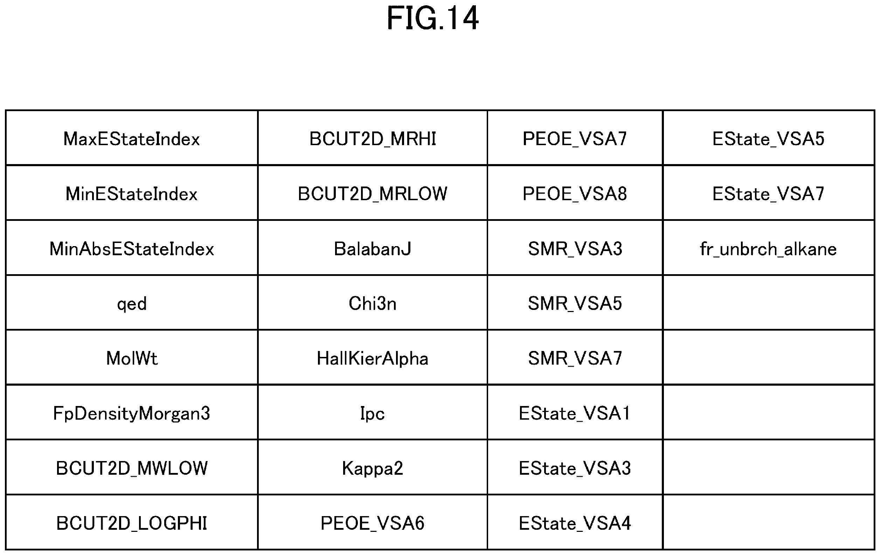

The learning process of generating the learned model 820 by using the training data was performed in the following procedure.
- The chemical structure information on the lipid molecule is converted into 209 molecular descriptors. FIG. 14 is a second diagram illustrating a specific example of the molecular descriptor.

- At this time, molecular descriptors having small variances are deleted from the 209 molecular descriptors.
- Additionally, molecular descriptors that are found to be collinear are deleted.
- The learning process is performed by inputting the molecular descriptor that is not deleted, as the input data, and the PK data (the measured value), as the correct data, into the learning model. As the learning model, a Regressor Chain (the base estimator is the random forest) is used.

### (3) Procedure for Verifying Inference Accuracy of Learned Model

The verification process of verifying the inference accuracy of the learned model 820 by using the verification data was performed in the following procedure.
- The chemical structure information on the lipid molecule is converted into the molecular descriptor used when the learned model 820 is generated.
- The converted molecular descriptor is input into the learned model 820 to infer the PK data.
- The inferred PK data and the measured PK data are compared to verify the inference accuracy of the learned model 820.

### (4) Verification Result

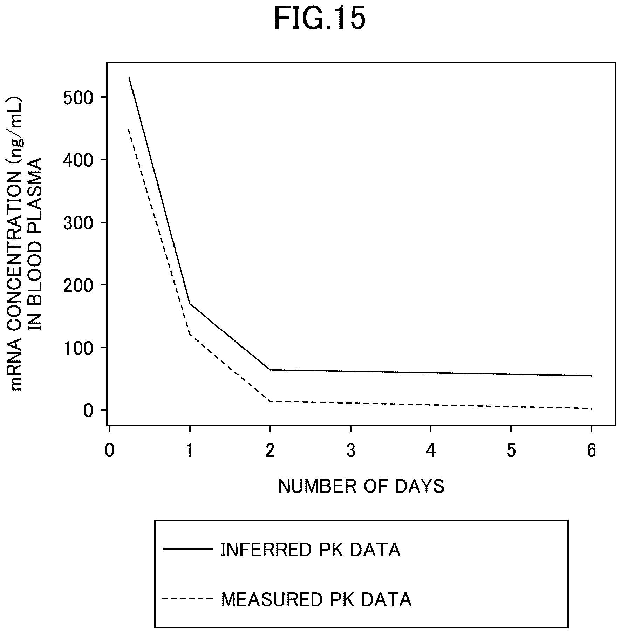

FIG. 15 is a second diagram indicating a specific example of the verification result. As indicated in FIG. 15, it has been verified that the PK data has a good inference accuracy.

### [Second Embodiment]

In the evaluation process of the first embodiment described above, when it is determined that the first evaluation data satisfies the predetermined condition in the first inference phase, the process proceeds to the second inference phase, and then it is determined whether the second evaluation data satisfies the predetermined condition. However, the processing procedure of the evaluation process is not limited thereto, and may be a processing procedure in which when it is determined that the second evaluation data satisfies the predetermined condition in the first inference phase, the process proceeds to the second inference phase, and then it is determined whether the first evaluation data satisfies the predetermined condition.

Additionally, in the first embodiment described above, both the processing of determining whether the first evaluation data satisfies the predetermined condition and the processing of determining whether the second evaluation data satisfies the predetermined condition are performed. However, the processing content of the evaluation process is not limited thereto, and either the processing of determining whether the first evaluation data satisfies the predetermined condition or the processing of determining whether the second evaluation data satisfies the predetermined condition may be performed.

### [Third Embodiment]

In the first and second embodiments described above, the description assumes that the designer 120 designs or selects a chemical structure of a new lipid molecule based on the first evaluation data and/or the second evaluation data. However, the process of designing or selecting a chemical structure of a new lipid molecule may be performed by, for example, the generation device. In the following, a third embodiment will be described, focusing on the difference from the first and second embodiments.

### <Outline of Process in Evaluation System>

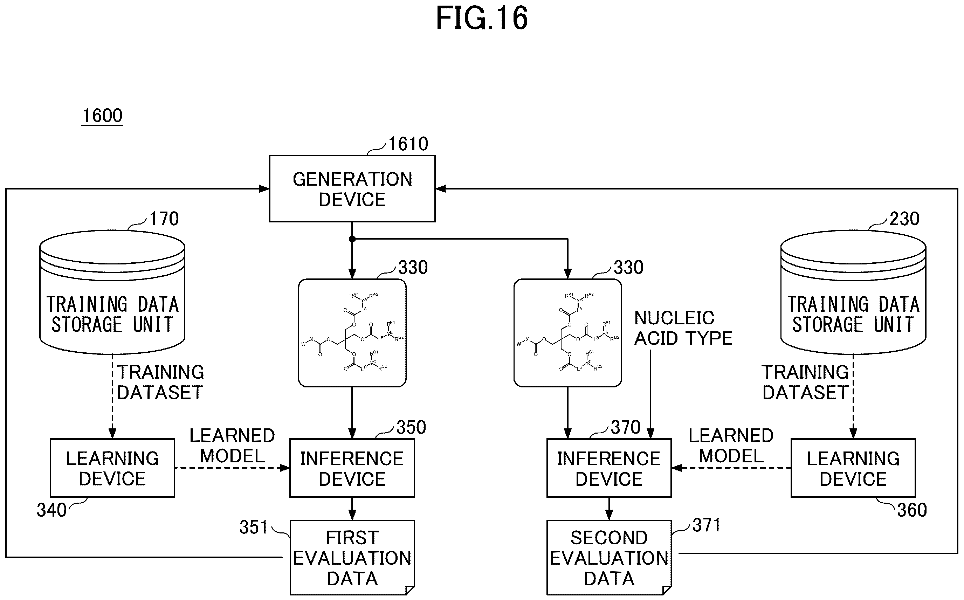

First, an outline of a process in the evaluation system according to the third embodiment will be described. FIG. 16 is a second diagram for explaining the outline of the process in the evaluation system. The difference from the evaluation system 100 illustrated in FIG. 3 is that, in an evaluation system 1600 illustrated in FIG. 16, a generation device 1610 executes the generation program to newly design or select the chemical structure of the lipid molecule and output the chemical structure information 330, instead of the designer 120.

The generation device 1610 has, for example, a reinforcement learning function by Thompson Sampling. Specifically, the generation device 1610 determines whether the first evaluation data 351 satisfies a predetermined condition and the second evaluation data 371 satisfies a predetermined condition.

If it is determined that the predetermined condition is not satisfied, the generation device 1610 generates the chemical structure information 330 on the lipid molecule based on the first evaluation data and the second evaluation data. Additionally, the generation device 1610 notifies the inference devices 350 and 370 of the generated chemical structure information 330 on the lipid molecule.

If it is determined that the first evaluation data 351 satisfies the predetermined condition and it is determined that the second evaluation data 371 satisfies the predetermined condition, the generation device 1610 outputs the chemical structure information on the corresponding lipid molecule as a search result. As described, the generation device 1610 repeatedly performs the processing of generating the chemical structure information on the new lipid molecule until it is determined that the predetermined condition is satisfied.

### <Flow of Evaluation Process>

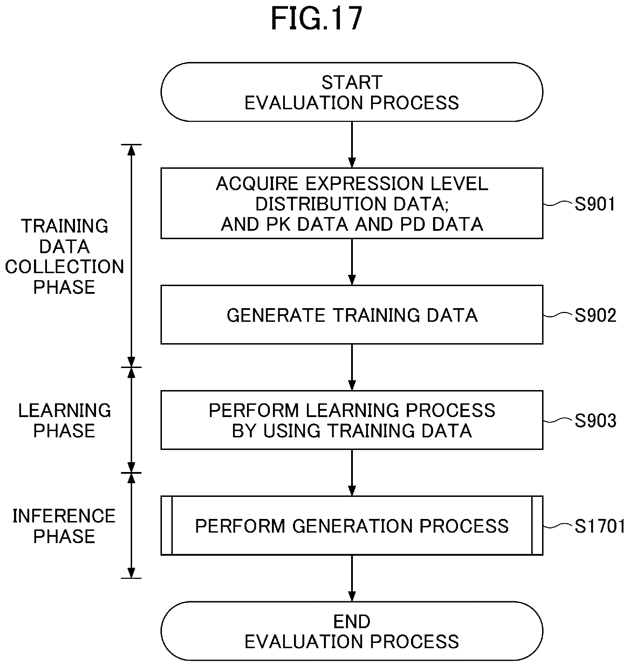

Next, a flow of an evaluation process by the evaluation system 1600 according to the third embodiment will be described. FIG. 17 is a second flowchart illustrating the flow of the evaluation process. As illustrated in FIG. 17, the evaluation process includes a training data collection phase, a learning phase, and an inference phase.

Among these, steps S901 to S902 of the training data collection phase and step S903 of the learning phase are the same as steps S901 to S903 in FIG. 9, and thus description thereof will be omitted here.

In step S1701 of the inference phase, the generation device 1610 performs a generation process. Here, the generation process of the generation device 1610 will be described in detail below.

### <Details of Generation Process>

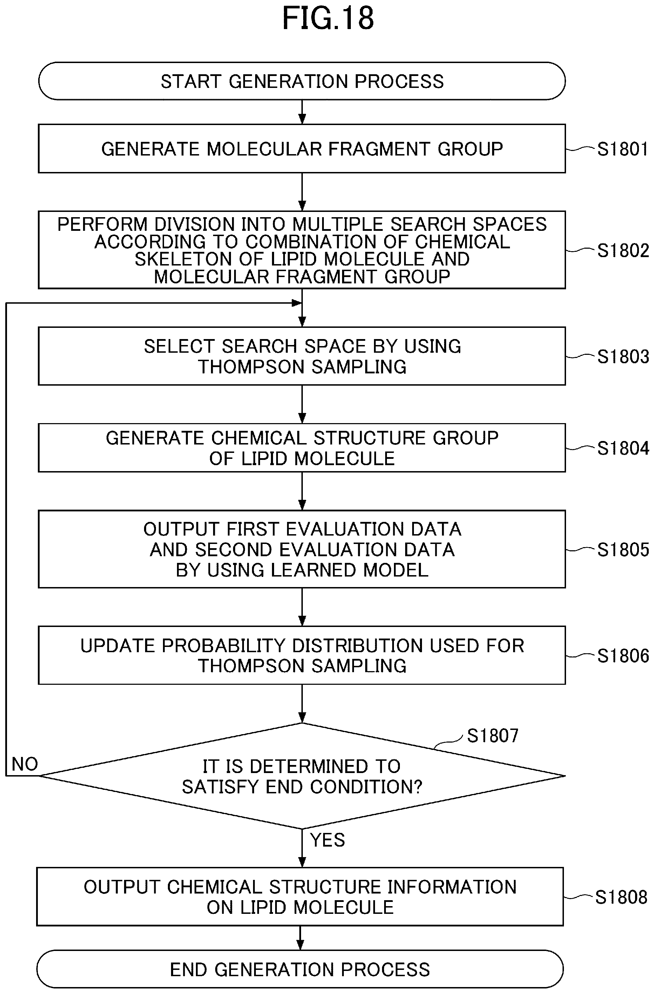

FIG. 18 is an example of a flowchart illustrating a flow of the generation process.

In step S1801, the generation device 1610 generates a molecular fragment group from a chemically formable hydrocarbon, in which the maximum values of the length, the saturation, and the number of branches are set.

In step S1802, the generation device 1610 combines the generated molecular fragments with a chemical skeleton of the lipid selected by the designer 120 to generate a chemical structure group of the lipid molecule. At this time, the generation device 1610 divides the generated chemical structure group of the lipid molecule into multiple search spaces according to the combination of the length, the degree of saturation, and the number of branches of the molecular fragment, the type of the chemical skeleton, and the like. The number of the multiple search spaces is specified by the designer 120.

In step S1803, the generation device 1610 selects a search space from among the multiple search spaces obtained by the dividing in step S1802 by using Thompson Sampling. Selecting the search space is nothing but selecting the characteristics of the search space (the combination of the length, the degree of saturation, the number of branches of the molecular fragment of the lipid molecule, the type of the chemical skeleton, and the like).

In step S1804, the generation device 1610 generates a chemical structural group of the lipid molecule by using the selected combination of the length, the degree of saturation, and the number of branches of the molecular fragment of the lipid molecule, the type of the chemical skeleton, and the like. Here, the generation device 1610 generates the chemical structure group of the lipid molecule by randomly acquiring multiple molecular fragments from a search space other than the selected search space with a certain probability, together with the selected molecular fragments.

In step S1805, the generation device 1610 notifies the inference devices 350 and 370 of respective chemical structure information on the generated chemical structure group of the lipid molecule. Additionally, the inference device 350 and the inference device 370 input the respective chemical structure information on the lipid molecule notified from the generation device 1610 into the learned models 620 and 820. With this, the inference device 350 and the inference device 370 output the first evaluation data and the second evaluation data.

In step S1806, the generation device 1610 updates probability distribution used for Thompson Sampling by using:
- the first evaluation data output by the inference device 350;
- the second evaluation data output by the inference device 370; and
- the search space selected in step S1803.

In step S1807, the generation device 1610 determines whether the first evaluation data and the second evaluation data satisfy a predetermined condition. If it is determined in step S1807 that either the first evaluation data or the second evaluation data does not satisfy the predetermined condition (NO in step S1807), the process returns to step S1803.

If it is determined in step S1807 that the first evaluation data satisfies the predetermined condition and the second evaluation data satisfies the predetermined condition (YES in step S1807), the process proceeds to step S1808.

In step S1808, the generation device 1610 outputs the corresponding chemical structural information on the lipid molecul as a search result.

### <Summary>

As is clear from the above description, the evaluation system 1600 according to the third embodiment is configured as follows.
- The preprocessing unit 610 or the preprocessing unit 810 acquires the input data including at least the chemical structure information on the lipid molecule.
- The learned model 620 that associates the input data including at least the chemical structure information on the lipid molecule with the information (the expression level distribution data at each time) indicating the distribution or behavior of the active ingredient encapsulated in the particle containing the lipid molecule in the living organism is generated. Alternatively, the learned model 820 that associates the input data including at least the chemical structure information on the lipid molecule with the information (the PK data and PD data) indicating the distribution or behavior of the active ingredient encapsulated in the particle containing the lipid molecule in the living organism is generated.
- The input data newly acquired by the preprocessing unit 610 is input into the learned model 620, and the information (the expression level distribution data at each time) indicating the distribution or behavior in the living organism that is associated with the newly acquired input data is inferred. Alternatively, the input data newly acquired by the preprocessing unit 810 is input into the learned model 820, and the information (PK data and PD data) indicating the distribution or behavior in the living organism that is associated with the newly acquired input data is inferred.
- The information indicating the distribution or behavior in the predetermined tissue and/or cell (the change over time in the expression level in the designated organ) is output based on the inferred information indicating the distribution or behavior in the living organism (the expression level distribution data at each time).
- The information (the PK feature, the PD feature, the PK/PD feature) indicating the behavior in any one or more of the predetermined tissue, blood, or urine is output based on the inferred information (the PK data and the PD data) indicating the distribution or behavior in the living organism.
- The generation device 1610 generates the chemical structure of the next new lipid molecule until the first evaluation data and the second evaluation data satisfy the predetermined condition.

With this, according to the generation device 1610 of the third embodiment, the chemical structure information on the lipid molecule that outputs the first evaluation data and the second evaluation data satisfying the predetermined condition can be generated. That is, according to the evaluation system 1800 of the third embodiment, the operation of designing or selecting the chemical structure of the lipid molecule forming the particle encapsulating the active ingredient can be supported.

### <Example 3>

Next, an example where the evaluation system 1600 according to the third embodiment is used will be described. Here, an example where chemical structure information on the lipid molecule is generated using a learned model generated by a generation procedure substantially the same as the generation procedure of the learned model described in the first embodiment will be described.
(1) Generation Procedure of Chemical Structure Information on Lipid Molecule
   - Information on the target organ (liver in this case) selected by the designer 120, the volume of the active molecules, the buffer composition in the particle, and the particle diameter is input to the generation device 1610.
   - The generation device 1610 is operated to generate the molecular fragment group from a chemically formable hydrocarbon, in which the maximum values of the length, the degree of saturation, and the number of branches are set.
   - The generated molecular fragment group and the chemical skeleton of the lipid molecule selected by the designer 120 are combined to generate the chemical structure group of the lipid molecule.
      a) At this time, the generation device 1610 divides the search space into the number defined by the designer 120 according to the length, the degree of saturation, and the number of branches of the molecular fragment and the type of the chemical skeleton.
      b) Subsequently, the generation device 1610 acquires the length, the degree of saturation, the number of branches of the molecular fragment of the lipid molecule, and the type of chemical skeleton to be generated from the search space divided in a) above by using Thompson Sampling.
      c) Subsequently, the generation device 1610 generates the chemical structure group of the lipid molecule by using the length, the degree of saturation, and the number of branches of the molecular fragment of the lipid molecule and the type of the chemical skeleton that are acquired. Here, the generation device 1610 generates the chemical structure group of the lipid molecule by randomly acquiring multiple molecular fragments with a certain probability from a search space other than the search space acquired in b) above, together with the molecular fragments acquired in b) above.
      d) Subsequently, the generation device 1610 infers the expression level distribution data (the probability of the expression in each organ) by using the learned model 620 with respect to the chemical structure group of the lipid molecule generated in c) above.
      e) Subsequently, the generation device 1610 updates the probability distribution used for Thompson Sampling by using the maximum value of the expression level distribution data inferred in the above d) and the search space acquired in the above b) .
      f) The generation device 1610 repeats a) to e) described above until expression level prediction data and the chemical structure group of the lipid molecule converge.
(2) Specific Example of Generated Chemical Structure Information on Lipid Molecule

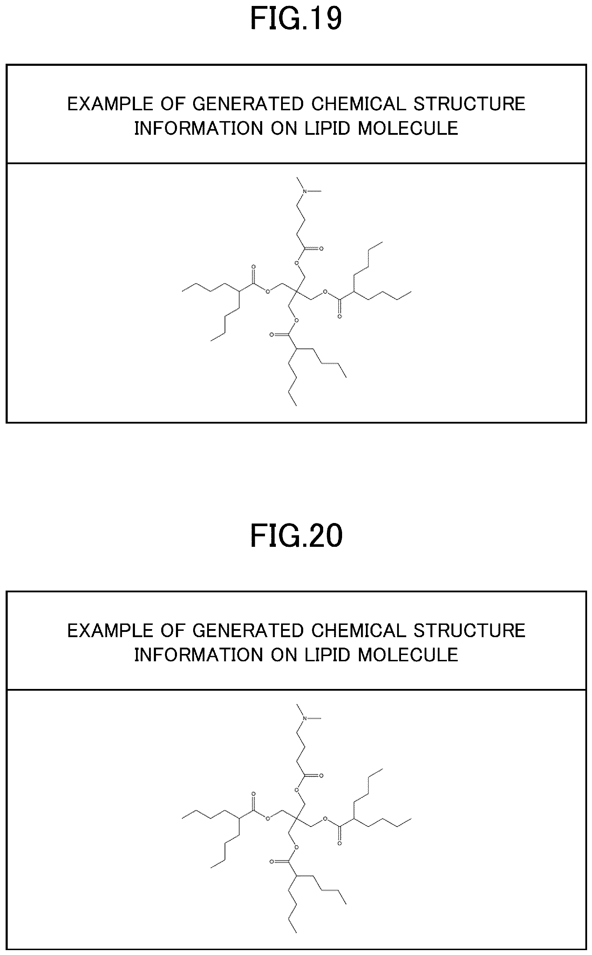

FIG. 19 is a first diagram illustrating a specific example of the generated chemical structure information on the lipid molecule. Here, it is confirmed that the lipid particle containing the lipid molecule having the chemical structure information illustrated in FIG. 19 is distributed in the liver. That is, it is verified that the generation device 1610 can generate the chemical structure information on the lipid molecule suitable for the target organ selected by the designer 120.

### <Example 4>

Next, an example where the evaluation system 1600 according to the third embodiment is used will be described. Here, an example where the chemical structure information on the lipid molecule is generated using a learned model described in a generation procedure substantially the same as the generation procedure of the learned model described in the second embodiment will be described.
(1) Generation Procedure of Chemical Structure Information on Lipid Molecule
   - The PK data suitably selected by the designer 120 is input to the generation device 1610.
   - The generation device 1610 is operated to generate the molecular fragment group from a chemically formable hydrocarbon, in which the maximum values of the length, the degree of saturation, and the number of branches are set.
   - The generated molecular fragment group is combined with the chemical skeleton of the lipid molecule selected by the designer 120 to generate the chemical structure group of the lipid molecule.
      a) At this time, the generation device 1610 divides the search space into the number defined by the designer 120 according to the length, the degree of saturation, and the number of branches of the molecular fragment and the type of the chemical skeleton.
      b) Subsequently, the generation device 1610 acquires the length, the degree of saturation, the number of branches of the molecular fragment of the lipid molecule to be generated, and the type of chemical skeleton of the lipid molecule to be generated from the search space divided in a) above by using Thompson Sampling.
      c) Subsequently, the generation device 1610 generates the chemical structure group of the lipid molecule by using the length, the degree of saturation, and the number of branches of the molecular fragment of the lipid molecule and the type of the chemical skeleton that are acquired. Here, the generation device 1610 generates the chemical structure group of the lipid molecule by randomly acquiring multiple molecular fragments with a certain probability from a search space other than the search space acquired in b) above, together with the molecular fragment acquired in b) above.
      d) Subsequently, the generation device 1610 infers the PK data by using the learned model 820 with respect to the chemical structure group of lipid molecule generated in the above c).
      e) Subsequently, the generation device 1610 calculates the similarity between the PK data inferred in the above d) and the PK data suitably selected by the designer 120 by using Dynamic Time Warping.
      f) Subsequently, the generation device 1610 updates the probability distribution used for Thompson Sampling by using a value at which the similarity calculated in e) above is maximized and the search space acquired in b) above.
      g) The generation device 1610 repeats a) to f) described above until the PK data and the chemical structure group of the lipid molecule converge.
(2) Specific Example of Generated Chemical Structure Information on Lipid Molecule

FIG. 20 is a second diagram illustrating a specific example of the generated chemical structure information on the lipid molecule. As illustrated in FIG. 20, it is confirmed that the similarity between the PK data selected by the designer 120 and the measured PK data is high according to the generation device 1610. That is, it is verified that the generation device 1610 can generate the chemical structure information on the lipid molecule having the PK data selected by the designer 120.

### [Fourth Embodiment]

In the third embodiment described above, no constraint is particularly applied when the generation device 1610 generates the chemical structure information on the lipid molecule. However, the generation device 1610 may be configured to generate the chemical structure information on the lipid molecule under a predetermined constraint.

Specifically, the generation device 1610 may be provided with a condition acquisition unit configured to acquire a predetermined constraint condition for designing or selecting the chemical structure of the lipid molecule forming the particle encapsulating the active ingredient. Additionally, the generation device 1610 may be configured to generate the chemical structure information on the lipid molecule under the predetermined constraint condition acquired by the condition acquisition unit. At this time, the predetermined constraint condition acquired by the condition acquisition unit may be, for example, a design precondition 310.

### [Fifth Embodiment]

In the first to fourth embodiments described above, the description assumes that the inference device 350 outputs the expression level distribution data of the designated organ at each time (the change over time in the expression level in the designated organ) as the first evaluation data 351. However, the output content of the first evaluation data is not limited thereto. For example, the mass distribution data of the nucleic acid of the designated organ at each time may be output. In the following, a fifth embodiment will be described, focusing on the differences from the first to fourth embodiments.

### <Functional Configuration of Inference Device>

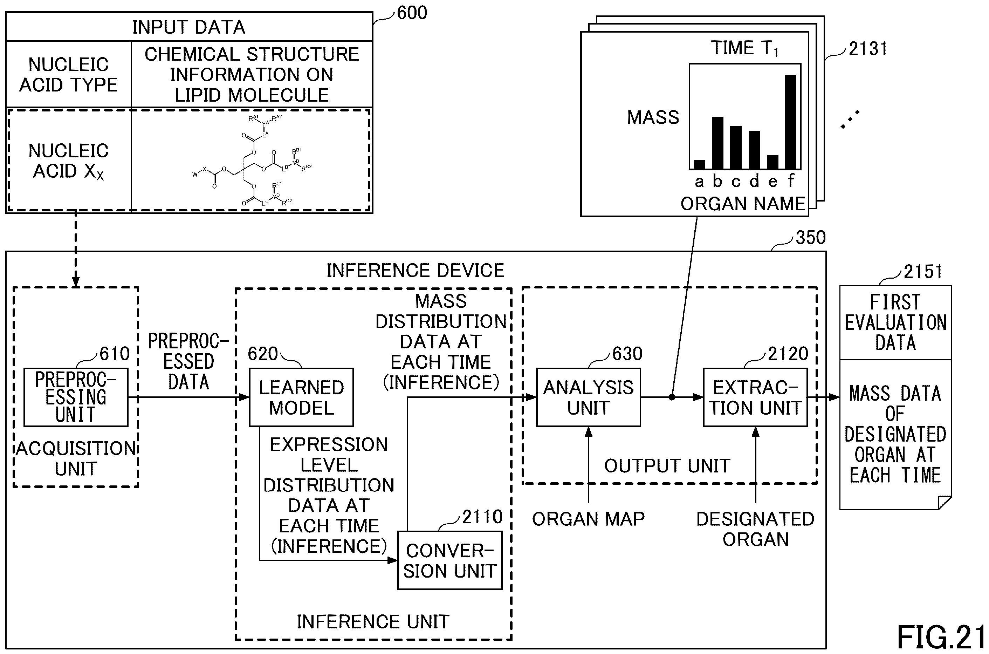

First, a functional configuration of an inference device according to the fifth embodiment will be described. FIG. 21 is a third diagram illustrating an example of a functional configuration of the inference device.

The difference from the functional configuration of the inference device 350 illustrated in FIG. 6 is that the inference device 350 illustrated in FIG. 21 includes a conversion unit 2110 and includes an extraction unit 2120 instead of the extraction unit 640.

The conversion unit 2110 with the learned model 620 is an example of the inference unit, and acquires the expression level distribution data at each time inferred by the learned model 620, and converts the acquired expression level distribution data at each time into mass distribution data at each time. Here, it is assumed that the expression level distribution data at each time and the mass distribution data at each time can be converted on a one-to-one basis. Therefore, as indicated by the reference numeral 2131, the analysis unit 630 can calculate the mass of the nucleic acid in each organ at each time.

The extraction unit 2120 with the analysis unit 630 is an example of the output unit, and outputs the mass of the nucleic acid in the designated organ. Specifically, the extraction unit 2120 extracts the mass of the nucleic acid in the designated organ from the mass of the nucleic acid calculated by the analysis unit 630 for each organ. Additionally, the extraction unit 2120 notifies the designer 120 of the extracted mass data of the designated organ at each time (the change over time in the mass of the nucleic acid in the designated organ) as first evaluation data 2151. That is, the first evaluation data 2151 is the information indicating the distribution or behavior of the specific tissue and/or cell in the target animal or the like 150. Here, the extraction unit 2120 may interpolate mass data between the times by using the extracted mass data of the designated organ at each time and notify the designer 120 of the first evaluation data 2151.

### <Summary>

As is clear from the above description, according to the fourth embodiment, the inference device 350 can output the mass data of the designated organ at each time (the change over time in the mass of the nucleic acid in the designated organ) as the first evaluation data instead of the expression level data of the designated organ at each time.

### [Sixth Embodiment]

In the fifth embodiment described above, the case where the mass distribution data at each time is acquired by converting the expression level distribution data at each time has been described. However, the method of acquiring the mass distribution data at each time is not limited thereto. For example, the mass distribution data at each time may be acquired by capturing using the fluorescence imaging system 160. In the following, a sixth embodiment will be described, focusing on the difference from the first to fifth embodiments.

### <Collection Example of Mass Distribution Data>

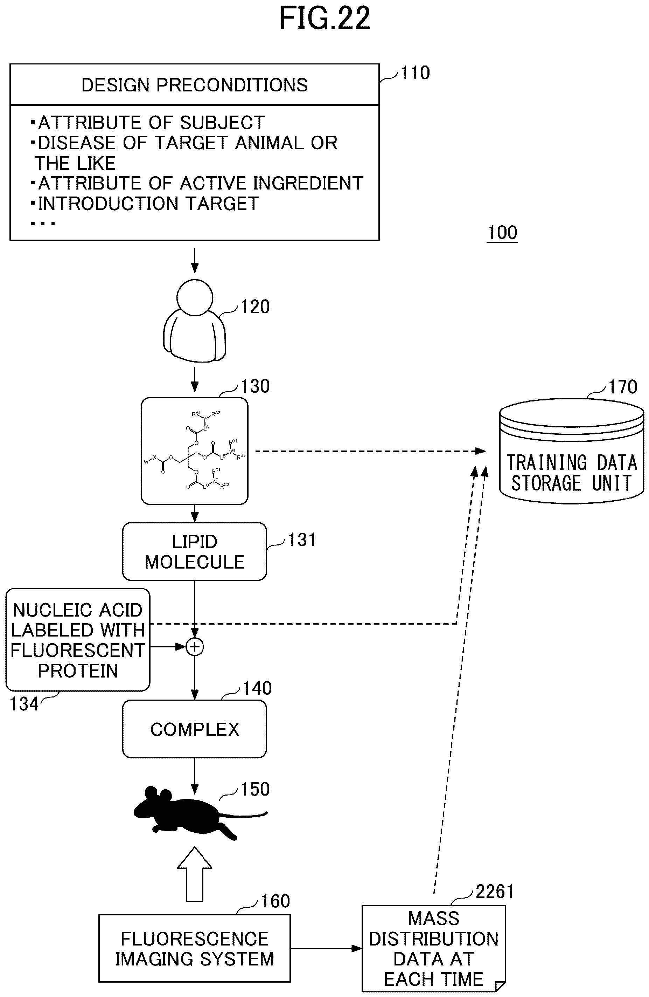

First, a collection example of "mass distribution data at each time" collected as the training data in the evaluation system according to the sixth embodiment will be described. FIG. 22 is a diagram illustrating a collection example of the mass distribution data. The difference from FIG. 1 is that a nucleic acid 134 labeled with a fluorescent protein is encapsulated in the particle containing the lipid molecule 131, and the particle of the complex 140 is formed. Additionally, the difference from FIG. 1 is that the fluorescence imaging system 160 captures fluorescence intensity distribution data indicating the mass of the active ingredient at each position and at each time in the target animal or the like 150.

As illustrated in FIG. 22, when the particle of the complex 140 is administered to the target animal or the like 150, the fluorescence imaging system 160 captures the fluorescence intensity distribution data indicating the mass of the active ingredient at each position and at each time in the target animal or the like 150 at predetermined time intervals. Additionally, the fluorescence imaging system 160 outputs the captured fluorescence intensity distribution data at each time as mass distribution data 1461 at each time.

Here, the mass distribution data 2261 at each time, output from the fluorescence imaging system 160, is stored in the training data storage unit 170 as the training data together with the chemical structure information 130 of the corresponding lipid molecule and the type of the corresponding active ingredient.

Here, the above-described collection method of the mass distribution data is an example, and the mass distribution data may be collected by another collection method. For example, the mass distribution data can also be acquired by administering the particle of the complex 140 to the living organism of the target animal or the like 150, then extracting each tissue or cell from the living organism at a certain time, and measuring the mass of the nucleic acid in each tissue or cell. According to this method, the mass distribution data can be acquired without using the fluorescent label of the nucleic acid encapsulated in the particle of the complex 140 and the fluorescence intensity distribution data.

### <Functional Configuration of Learning Device>

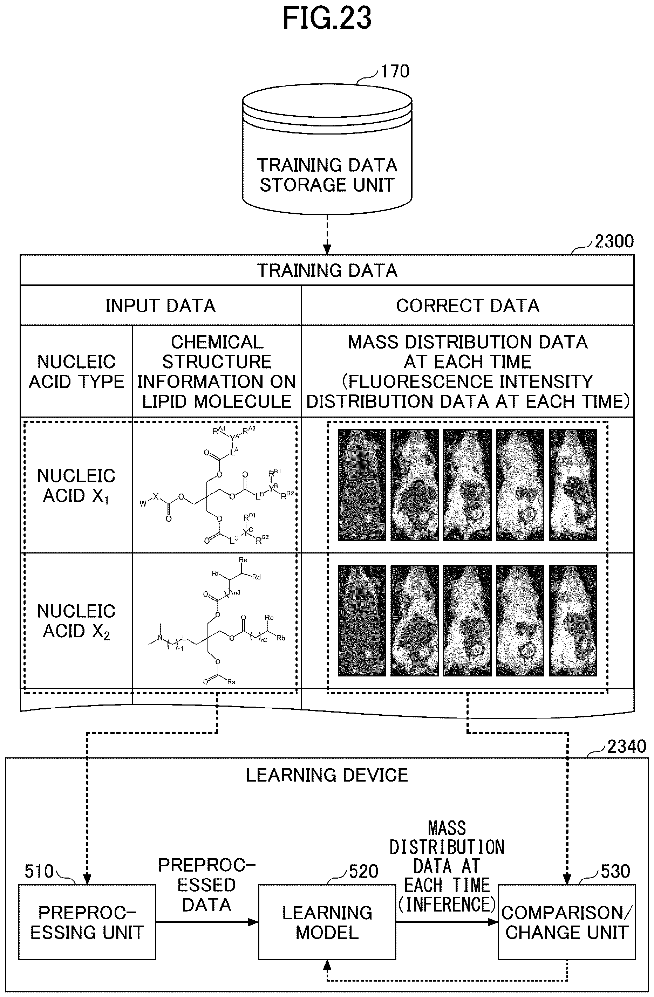

Next, a functional configuration of the learning device will be described. FIG. 23 is a third diagram illustrating an example of the functional configuration of the learning device. In FIG. 23, training data 2300 is an example of the training data that is generated based on the mass distribution data 2261 at each time and that is stored in the training data storage unit 170. Here, although items of each information of the training data (input data and correct data) are exemplified below, the items are not limited thereto, and another item may be used as the training data, or some of these items may be used as the training data.

As illustrated in FIG. 23, the training data 2300 includes input data and correct data, and the input data includes "nucleic acid type" and "chemical structure information on lipid molecule" as the items of the information. Additionally, the correct data includes "mass distribution data at each time (fluorescence intensity distribution data at each time)".

Among these, the "nucleic acid type" and the "chemical structure information on lipid molecule" of the input data have been described in the first embodiment described above with reference to FIG. 5, and thus the description thereof will be omitted here.

"mass distribution data at each time (fluorescence intensity distribution data at each time)" stores an image representing the mass of the nucleic acid at each position in the living organism at each time, after the particle of the complex in which the corresponding nucleic acid (the nucleic acid labeled with a fluorescent protein) is encapsulated in the particle containing the corresponding lipid molecule is administered to the living organism of the target animal or the like 150. The example of FIG. 23 illustrates a state in which mass distribution data at five different times are stored, and the difference in color for each pixel in each mass distribution data indicates that the mass of the active ingredient differs according to the position in the living organism, corresponding to the pixel. Additionally, the difference in the colored position, colored range, and color between the mass distribution data indicates that the mass, position, and range of the active ingredient in the living organism change as the time passes.

Here, these training data may be data obtained from various public information (for example, patent publications and papers) or databases.

### <Functional Configuration of Inference Device>

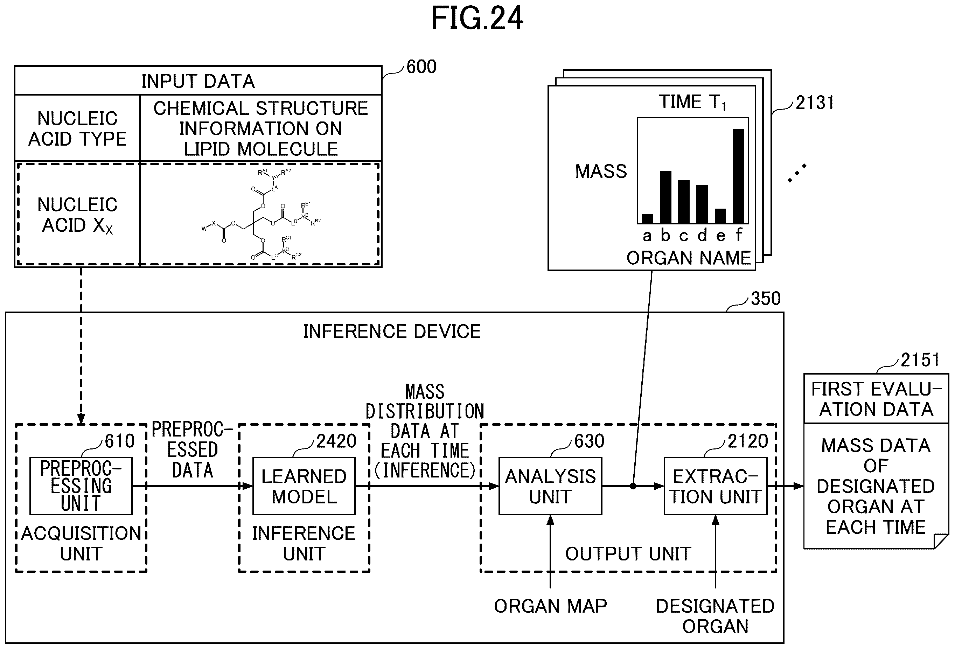

Next, a functional configuration of an inference device according to the sixth embodiment will be described in detail. FIG. 24 is a fourth diagram illustrating an example of the functional configuration of the inference device. The difference from the functional configuration of the inference device 350 illustrated in FIG. 21 is that a learned model 2420 is included instead of the learned model 620.

The learned model 2420 is generated by a learning device 2340 performing a learning process. In response to the preprocessed data notified from the preprocessing unit 610 being input, the learned model 2420 infers mass distribution data at each time.

### <Summary>

As is clear from the above description, according to the sixth embodiment, the inference device 350 can output the mass data of the designated organ at each time (a change over time in the mass of the nucleic acid in the designated organ) instead of the expression level data of the designated organ at each time as the first evaluation data.

### [Other Embodiments]

In the first to sixth embodiments described above, the description assumes that the learning device and the inference device are configured as separate devices. However, the learning device and the inference device may be configured as an integrated device.

Additionally, in the first to sixth embodiments described above, the description assumes that the learning device 340 and the learning device 360 are configured as separate devices. However, the learning device 340 and the learning device 360 may be configured as an integrated device. Similarly, in the first to sixth embodiments described above, the description assumes that the inference device 350 and the inference device 370 are configured as separate devices. However, the inference device 350 and the inference device 370 may be configured as an integrated device.

Additionally, in the first to sixth embodiments described above, the description assumes that the learning device and the inference device are implemented by a single computer. However, the learning device and the inference device may be implemented by multiple computers in a distributed computing mode.

Additionally, in the first to sixth embodiments described above, as an example of a method of installing the learning program or the inference program in the auxiliary storage device 403, a method of downloading the program via a network, which is not illustrated, and installing the program has been described. At this time, although the download source is not particularly described, in the case of installing the learning program or the inference program by such a method, the download source may be, for example, a server device in which the learning program or the inference program is stored in an accessible manner. Additionally, the server device may be, for example, a device that receives access from the learning device or the inference device via a network, which is not illustrated, and downloads the prediction program on the condition of charging. That is, the server device may be a device that performs a service of providing the learning program or the inference program on a cloud.

Additionally, in the fourth embodiment described above, the generation device 1610 is configured to output the search result when it is determined that the first evaluation data and the second evaluation data satisfy the predetermined condition. However, instead of determining whether the predetermined condition is satisfied, for example, it may be determined whether the chemical structure information on the lipid molecule to be generated has been updated. In this case, the generation device 1010 may be configured to output the search result when it is determined that the chemical structure information on the lipid molecule to be generated has not been updated.

Additionally, in each of the embodiments described above, details of the learning model are not described, but the learning model used in each of the embodiments described above may be a machine learning model including deep learning, and for example, any of the following models may be used:
- a recurrent neural network (RNN);
- a long short-term memory (LSTM);
- a convolutional neural network (CNN);
- a region based convolutional neural network (R-CNN) ;
- a generative adversarial network (GAN); and
- a support vector machine (SVM).

Here, the present invention is not limited to the configurations described herein, such as the configurations described in the embodiments, combinations with other elements, and the like. These points can be changed without departing from the scope of the present invention, and can be appropriately determined according to the application form thereof.

This application claims priority to Japanese Patent Application No. 2022-28996 filed on February 28, 2022, the entire contents of which are incorporated herein by reference.

### Description of reference numerals

100: evaluation system
160: fluorescence imaging system
161: expression level distribution data at each time
170: training data storage unit
220: PK/PD analysis system
221: PK data and PD data
340: learning device
350: inference device
351: first evaluation data
360: learning device
370: inference device
371: second evaluation data
500: training data
510: preprocessing unit
520: learning model
530: comparison/change unit
610: preprocessing unit
620: learned model
630: analysis unit
640: extraction unit
700: training data
710: preprocessing unit
720: learning model
730: comparison/change unit
810: preprocessing unit
820: learned model
830: feature calculation unit
840: calculation unit
850: feature calculation unit
1600: evaluation system
1610: generation device
2110: conversion unit
2120: extraction unit
2151: first evaluation data
2261: mass distribution data at each time
2300: training data
2340: learning device
2420: learned model
2440: extraction unit
2451: first evaluation data

## Claims

1. An inference device comprising:
an acquisition unit configured to acquire input data including at least chemical structure information on a lipid molecule;
an inference unit configured to input the input data newly acquired by the acquisition unit into a learned model generated by performing a learning process on a learning model that associates input data including at least chemical structure information on a lipid molecule with information indicating distribution or behavior of an active ingredient encapsulated in a particle containing the lipid molecule in a living organism, to infer information indicating distribution or behavior in the living organism, associated with the newly acquired input data; and
an output unit configured to output, based on the inferred information indicating the distribution or behavior in the living organism, information indicating distribution or behavior in a predetermined tissue and/or cell in the living organism or information indicating behavior in any one or more of tissue, blood, or urine.

2. The inference device as claimed in claim 1,
wherein the information indicating the distribution or behavior in the living organism includes mass distribution data or expression level distribution data of the active ingredient encapsulated in the particle containing the lipid molecule in the living organism at each time,
wherein the inference unit infers mass distribution data or expression level distribution data at each time, associated with the newly acquired input data, and
wherein the output unit outputs a change over time in mass or an expression level in the predetermined tissue and/or cell based on the inferred mass distribution data or expression level distribution data at each time.

3. The inference device as claimed in claim 2, wherein the expression level distribution data at each time used when the learning process is performed is fluorescence intensity distribution data at each time captured by introducing, into the living organism, a nucleic acid encoding a fluorescent protein, the nucleic acid being encapsulated in the particle containing the lipid molecule having the chemical structure information used when the learning process is performed.

4. The inference device as claimed in claim 3, wherein the mass distribution data at each time used when the learning process is performed is calculated based on the fluorescence intensity distribution data at each time.

5. The inference device as claimed in claim 2, wherein the mass distribution data at each time used when the learning process is performed is fluorescence intensity distribution data at each time captured by introducing, into the living organism, a nucleic acid labeled with a fluorescent protein, the nucleic acid being encapsulated in the particle containing the lipid molecule having the chemical structure information used when the learning process is performed.

6. The inference device as claimed in any one of claims 3 to 5, wherein the learned model is generated by updating model parameters of the learning model so that output data when the input data including at least the chemical structure information on the lipid molecule is input into the learning model approaches the fluorescence intensity distribution data at each time.

7. The inference device as claimed in claim 2, wherein the output unit calculates the expression level at each time in the predetermined tissue and/or cell in the living organism based on the inferred expression level distribution data at each time, and outputs the change over time in the expression level in the predetermined tissue and/or cell in the living organism by interpolating an expression level between times.

8. The inference device as claimed in claim 2, wherein the output unit calculates the mass at each time in the predetermined tissue and/or cell in the living organism based on the inferred mass distribution data at each time, and outputs the change over time in the mass in the predetermined tissue and/or cell in the living organism by interpolating mass between times.

9. The inference device as claimed in claim 1,
wherein the information indicating the distribution or behavior in the living organism includes PK data and/or PD data of the active ingredient encapsulated in the particle containing the lipid molecule,
wherein the inference unit infers PK data and/or PD data associated with the newly acquired input data, and
wherein the output unit outputs, based on the inferred PK data and/or PD data, a feature related to a change over time in any one or more of a tissue concentration, a blood concentration, or a urine concentration, and a feature indicating a relationship between any one or more of a tissue concentration, a blood concentration, or a urine concentration; and a pharmacological effect and/or toxicity.

10. The inference device as claimed in claim 9, wherein the output unit calculates PK/PD data based on the inferred PK data and PD data, and outputs a feature related to a change over time in the pharmacological effect and/or toxicity based on the calculated PK/PD data.

11. The inference device as claimed in claim 9, wherein the PK data and/or PD data used when the learning process is performed is PK data and/or PD data measured by introducing, into the living organism, the active ingredient encapsulated in the particle containing the lipid molecule having the chemical structure information used when the learning process is performed.

12. The inference device as claimed in claim 11, wherein the learned model is generated by updating model parameters of the learning model so that an output when the input data including at least the chemical structure information on the lipid molecule is input into the learned model approaches the measured PK data and/or PD data.

13. A generation device that repeats a generation process of generating chemical structure information on a new lipid molecule until the information indicating distribution or behavior in the predetermined tissue and/or cell in the living organism or the information indicating behavior in any one or more of the tissue, blood, or urine, output by the inference unit of the inference device as claimed in claim 1, satisfies a predetermined condition.

14. The generation device as claimed in claim 13, wherein the generation device selects a search space from among a plurality of search spaces obtained according to a combination of a molecular fragment of a formable hydrocarbon and a chemical skeleton of a lipid molecule, based on the information indicating the distribution or behavior in the predetermined tissue and/or cell in the living organism or the information indicating the behavior in any one or more of the tissue, blood, or urine, and generates chemical structure information on a next new lipid molecule by using a characteristic of the selected search space.

15. The generation device as claimed in claim 14, wherein the plurality of search spaces are different from each other in a combination of a length, a degree of saturation, and a number of branches of the molecular fragment and a type of the chemical skeleton of the lipid molecule.

16. The generation device as claimed in claim 14, wherein the generation device generates the chemical structure information on the next new lipid molecule under a predetermined constraint condition.

17. The generation device as claimed in claim 16, wherein the generation device generates the chemical structure information on the next new lipid molecule by using a precondition for designing or selecting a chemical structure of the lipid molecule forming the particle including the active ingredient as the predetermined constraint condition.

18. An inference program for causing a computer to execute:
an acquisition step of acquiring input data including at least chemical structure information on a lipid molecule;
an inference step of inputting the input data newly acquired in the acquisition step into a learned model generated by performing a learning process on a learning model that associates input data including at least chemical structure information on a lipid molecule with information indicating distribution or behavior of an active ingredient encapsulated in a particle containing the lipid molecule in a living organism, to infer information indicating distribution or behavior in the living organism, associated with the newly acquired input data; and
an output step of outputting, based on the inferred information indicating the distribution or behavior in the living organism, information indicating distribution or behavior in a predetermined tissue and/or cell in the living organism or information indicating behavior in any one or more of tissue, blood, or urine.

19. A generation program for causing a computer to execute a generation step of repeating a generation process of generating chemical structure information on a new lipid molecule until the information indicating the distribution or behavior in the predetermined tissue and/or cell in the living organism or the information indicating the behavior in any one or more of the tissue, blood, or urine, output by the inference unit of the inference device as claimed in claim 1, satisfies a predetermined condition.
